(19) **Europäisches Patentamt**
**European Patent Office**
**Office européen des brevets**

(11) **EP 2 167 687 B1**

(12) **EUROPEAN PATENT SPECIFICATION**

(45) Date of publication and mention
of the grant of the patent:
**27.03.2013 Bulletin 2013/13**

(21) Application number: **08770385.6**

(22) Date of filing: **06.06.2008**

(51) Int Cl.:
***C12Q 1/68*** (2006.01)

(86) International application number:
**PCT/US2008/066180**

(87) International publication number:
**WO 2008/154423 (18.12.2008 Gazette 2008/51)**

(54) **BIOMARKERS FOR PREDICTING ANTI-TNF RESPONSIVENESS OR NON-RESPONSIVENESS**

BIOMARKER ZUR VORHERSAGE DES ANSPRECHENS ODER NICHTANSPRECHENS AUF ANTI-TNF

BIOMARQUEURS POUR PRÉDIRE UNE RÉACTIVITÉ OU NON RÉACTIVITÉ ANTI-TNF

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **08.06.2007 US 942937 P**

(43) Date of publication of application:
**31.03.2010 Bulletin 2010/13**

(73) Proprietors:
• **Biogen Idec MA Inc.**
**Cambridge, MA 02142 (US)**
• **The Feinstein Institute for Medical Research**
**Manhasset, NY 11030 (US)**

(72) Inventors:
• **CARULLI, John, P.**
**Southborough, MA 01772 (US)**
• **GREGERSEN, Peter, K.**
**Larchmont, NY 10538 (US)**
• **BATLIWALLA, Franak**
**Port Washington, NY 11050 (US)**
• **BIENKOWSKA, Jadwiga**
**Cambridge, MA 02139 (US)**
• **LIU, Chunyu**
**Sharon, MA 02067 (US)**

(74) Representative: **Pohlman, Sandra M. et al**
**df-mp**
**Fünf Höfe**
**Theatinerstrasse 16**
**80333 München (DE)**

(56) References cited:
**WO-A2-2007/038501      WO-A2-2007/135568**
**WO-A2-2008/132176      US-A1- 2004 009 479**
**US-A1- 2007 105 133**

• **HUEBER ET AL: "Proteomic Signatures of Secreted Proteins for the Prediction of Treatment Response to TNF Blockers in RA" CLINICAL IMMUNOLOGY, ACADEMIC PRESS, US, vol. 123, 1 January 2007 (2007-01-01), page S94, XP022076744 ISSN: 1521-6616**
• **LEQUERRÉ THIERRY ET AL: "Gene profiling in white blood cells predicts infliximab responsiveness in rheumatoid arthritis" ARTHRITIS RESEARCH AND THERAPY, BIOMED CENTRAL, LONDON, GB LNKD- DOI: 10.1186/AR1924, vol. 8, no. 4, 3 July 2006 (2006-07-03), page R105, XP021020585 ISSN: 1478-6354**
• **KOCZAN DIRK ET AL: "Molecular discrimination of responders and nonresponders to anti-TNFalpha therapy in rheumatoid arthritis by etanercept" ARTHRITIS RESEARCH AND THERAPY, BIOMED CENTRAL, LONDON, GB, vol. 10, no. 3, 2 May 2008 (2008-05-02), page R50, XP021041217 ISSN: 1478-6354**
• **RISK ET AL.: 'Characterization of a 500 kb region on 17q25 and the exclusion of candidate genes as the familial Tylosis Oesophageal Cancer (TOC) locus' vol. 21, 2002, pages 6395 - 6402, XP008127232**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND**

Tumor Necrosis Factor-Alpha (TNF) is a cytokine that plays a key role in the pathogenesis of inflammatory diseases such as rheumatoid arthritis. Blockade of TNF signaling through the use of anti-TNF monoclonal antibodies (e.g., adalimumab or infliximab) or TNF receptor fusion proteins (e.g., etaneracept) can be used to ameliorate symptoms of certain inflammatory diseases. While anti-TNF treatments have been tremendously successful, they do not produce significant clinical responses in all patients who receive them.

Identification of patients as being likely or unlikely to respond to anti-TNF treatment is a critical for optimal patient management. Patients exhibiting biomarkers suggesting they are unlikely to respond to anti-TNF treatment can be steered to other therapies immediately (i.e., without administering an anti-TNF treatment) or upon failure with a first anti-TNF treatment.

**SUMMARY**

The present invention is based, at least in part, on the discovery of biomarkers that are predictive of a subject's responsiveness or non-responsiveness to an anti-TNF therapy. For example, the expression level of one or more of the genes depicted in Table 1 can predict the likelihood that a given subject will or will not respond to an anti-TNF therapy. The biomarkers, compositions, and methods described herein are thus useful in selecting appropriate treatment modalities (e.g., an anti-TNF therapy or a non-anti-TNF therapy) for a subject suffering from a disease such as an immune or inflammatory disorder (e.g., rheumatoid arthritis or Crohn's disease).

In one aspect, the disclosure provides an anti-TNF agent that inhibits the activity of TNF for use in treating an immune disorder in a subject, wherein the subject has been identified as having (a) an elevated expression level, as compared to a healthy individual of ANKIB1, or (b) an elevated expression level of ANKIB1 and in addition to ANKIB1, at least eight, at least nine, at least 10, at least 11, at least 12, at least 13, at least 14, at least 15, at least 16, at least 17, at least 18, at least 19, at least 20, at least 21, at least 22, or at least 24 further genes selected from the group consisting of (i) an elevated expression level, as compared to a healthy individual, of ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1, or (ii) a reduced expression level, as compared to a healthy individual, of ANKRD12, CAMK2G-, CASP5, CXorf52, DNAH1, EEA1, FAM44A, FOXJ3, HDAC4, MNT, MXRA7, PTCH1, SEL1L, or SFRS2.

Herein described is a method of treating an immune disorder, which method includes the step of administering to a subject in need thereof an effective amount of a therapy comprising a non-anti-TNF agent, wherein the subject has been identified as having at least one (e.g., at least two, at least three, at least four, at least five, at least six, at least seven, at least eight, at least nine, at least 10, at least 11, at least 12, at least 13, at least 14, at least 15, at least 16, at least 17, at least 18, at least 19, at least 20, at least 21, at least 22, or at least 24) of (i) an elevated expression level, as compared to a healthy individual, of one or more of ANKRD12, CAMK2G-, CLASP5, CXorf52, DNAH1, EEA1, FAM44A, FOXJ3, HDAC4, MNT, MXRA7, PTCH1, SEL1L, or SFRS2, or (ii) a reduced expression level, as compared to a healthy individual, of one or more of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG 1, YIPF6, ZNF294, or ZFP36L1.

In some embodiments, the subject has been identified as having elevated or reduced expression levels, as compared to a healthy individual, of at least five, at least six, at least seven, at least eight, at least nine, at least 10, at least 11, at least 12, at least 13, at least 14, at least 15, at least 16, at least 17, at least 18, at least 19, at least 20, at least 21, at least 22, at least 23, or at least 24 or more genes, including ANKIB1, wherein the additional genes are selected from the group consisting of ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2. In addition to ANKIB1, the at least five genes can include, e.g., ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6. The at least five genes can include, e.g., CLTB, COL4A3BP, CXorf52, FAM44A, MXRA7, PGK1, SFRS2, or YIPF6. In addition to ANKIB1, the at least eight genes can include, e.g., Arc 1 , ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and/or YIPF6. In addition to ANKIB1, the at least eight genes can include, e.g., CLTB, COL4A3BP, CXorf52, FAM44A, MXRA7, PGK1, SFRS2, and/or YIPF6. In addition to ANKIB1, the at least 24 genes can include, e.g., ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SxELIL, SERF2, SFRS2, and YIPF6.

In another aspect, the disclosure features a method of predicting the response of a subject to a therapy comprising an anti-TNF agent. The method includes the steps of: providing a biological sample obtained from a subject that has an immune disorder; and measuring the expression level of ANKIB1 in the biological sample, wherein (i) an elevated expression level, as compared to a healthy individual, of ANKIB1, predicts that the subject will respond to a therapy comprising an anti-TNF agent.

In one embodiment, in addition to measuring an elevated expression level of ANKIB1, the method includes measuring the expression level of one or more additional genes, wherein (i) an elevated expression level, as compared to a healthy individual, of ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1, or (ii) a reduced expression level, as compared to a healthy individual, of one or more of ANKRD12, CAMK2G-, CASP5, CXorf52, DNAH1, EEA1, FAM44A, FOXJ3, HDAC4, MNT, MXRA7, PTCH1, SEL1L, or SFRS2 predicts that the subject will respond to a therapy comprising an anti-TNF agent. The RNA or protein expression of the one or more genes can be measured. The RNA expression level of the one or more genes can be measured using, e.g., microarray analysis and/or quantitative polymerase chain reaction.

In some embodiments, the method includes determining that the expression level of ANKIB1 and one or more of (i) ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are elevated, as compared to a healthy individual, or (ii) ANKRD12, CAMK2G-, CASP5, CXorf52, DNAH1, EEA1, FAM44A, FOXJ3, HDAC4, MNT, MXRA7, PTCH1, SEL1L, or SFRS2 are reduced, as compared to a healthy individual; and selecting a therapy comprising an anti-TNF agent for the subject. The method can further include administering the therapy comprising an anti-TNF agent to the subject.

In some embodiments, the method can further include creating a record indicating that the subject is likely to respond to a therapy comprising an anti-TNF agent, if the expression level of ANKIB1 and one or more of(i) ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are elevated, as compared to a healthy individual, or (ii) ANKRD12, CAMK2G-, CASP5, CXorf52, DNAH1, EEA1, FAM44A, FOXJ3, HDAC4, MNT, MXRA7, PTCH1, SEL1L, or SFRS2 are reduced, as compared to a healthy individual. The record can be created, e.g., on a computer readable medium.

In some embodiments, the method can include determining that the expression level of ANKIB1 and one or more of (i) ANKRD12, CAMK2G-, CASP5, CXorf52, DNAH1, EEA1, FAM44A, FOXJ3, HDAC4, MNT, MXRA7, PTCH1, SEL1L, or SFRS2 are elevated, as compared to a healthy individual, or (ii) ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are reduced, as compared to a healthy individual; and selecting a therapy comprising a non-anti-TNF agent for the subject. The method can further include administering the therapy comprising a non-anti-TNF agent to the subject.

In some embodiments, the method can further include creating a record indicating that the subject is not likely to respond to a therapy comprising an anti-TNF agent, if the expression level of one or more of (i) ANKRD12, CAMK2G-, CASP5, CXorf52, DNAH1, EEA1, FAM44A, FOXJ3, HDAC4, MNT, MXRA7, PTCH1, SEL1L, or SFRS2 are elevated, as compared to a healthy individual, or (ii) ANKIB1 and one or more of ARF 1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are reduced, as compared to a healthy individual. The record can be created, e.g., on a computer readable medium.

In some embodiments, the expression level of at least (i) ANKIB1, or (ii) ANKIB1 and additionally at least one gene selected from the group consisting of ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2 is elevated or reduced, as compared to a healthy individual, by at least about 1.5 fold (e.g., at least about 2 fold, at least about 2.5 fold, at least about 3.0 fold, at least about 3.5 fold, at least about 4.0 fold, or at least about 5 fold or more). The methods described herein can include measuring the expression level of at least five, at least six, at least seven, at least eight, at least nine, at least 10, at least 11, at least 12, at least 13, at least 14, at least 15, at least 16, at least 17, at least 18, at least 19, at least 20, at least 21, at least 22, or at least 24 genes selected from ANKIB1 and one or more further genes selected from the group consisting of ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2. In addition to ANKIB1, the at least five genes can include, e.g., ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6. In addition to ANKIB1, the at least five genes can include, e.g., CLTB, COL4A3BP, CXorf52, FAM44A, MXRA7, PGK1, SFRS2, or YIPF6. In addition to ANKIB1, the at least eight genes can include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and/or YIPF6. The at least eight genes can include, e.g., CLTB, COL4A3BP, CXorf52, FAM44A, MXRA7, PGK1, SFRS2, and/or YIPF6. In addition to ANKIB1, the at least 24 genes can include, e.g., ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6.

In another aspect, the disclosure provides a method of predicting the response of a subject to therapy comprising an anti-TNF agent, which method includes the step of assessing the expression level (e.g., the RNA or protein expression level) of one or more genes in a biological sample from a subject, wherein a reduced expression level, as compared to a healthy individual, of ANKIB1 predicts that the subject will not respond to a therapy comprising an anti-TNF agent.

In one embodiment, in addition to a reduced expression level of ANKIB1, the method includes measuring the expression level of one or more additional genes wherein (i) an elevated expression level as compared to a healthy individual, of ANKRD12, CAMK2G-, CASP5, CXorf52, DNAH1, EEA1, FAM44A, FOXJ3, HDAC4, MNT, MXRA7, PTCH1, SEL1L, or SFRS2 or (ii) a reduced expression level, as compared to a healthy individual, of ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 predicts that the subject will not respond to an anti-TNF therapy.

In some embodiments, the method can include determining that the expression level of eight or more, nine or more, 10 or more, 11 or more, 12 or more, 13 or more, 14 or more, 15 or more, 16 or more, 17 or more, 18 or more, 19 or more, 20 or more, 21 or more, or 22 or more genes are elevated or reduced, wherein, in addition to a reduced expression of ANKIB1, ANKRD12, CAMK2G-, CASP5, CXorf52, DNAH1, EEA1, FAM44A, FOXJ3, HDAC4, MNT, MXRA7, PTCH 1, SEL1L, or SFRS2 are elevated or ARF 1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are reduced; and predicting that the subject will not respond to an anti-TNF therapy. In some embodiments, the methods include, after predicting that the subject will not respond to the anti-TNF therapy, administering to the subject a non-anti-TNF therapy.

Any of the uses described herein can further include a step of prescribing a therapy comprising an anti-TNF agent or non-anti-TNF agent (the choice of which depends upon the outcome of the predictive methods described herein) for the subject.

The biological sample in any of the uses described herein can consist of, or contain, e.g., blood.

In some embodiments, the subject can have a disease such as an immune (e.g., an inflammatory) disorder, an infection, or any disease treatable by a therapy comprising an anti-TNF agent described herein. For example, the subject can have rheumatoid arthritis or Crohn's disease. The subject can be a human.

In some uses, the anti-TNF agent can consist of, or contain, an anti-TNF antibody or a soluble TNF receptor. The anti-TNF antibody can be, e.g., adalimumab or infliximab. The soluble TNF receptor can be, e.g., etanercept.

As described herein, the non-anti-TNF agent can consist of, or contain, a non-steroidal anti-inflammatory drug (NSAID), a corticosteroid, a disease-modifying antirheumatic drug (DMARD), an anti-CD20 antibody, a TWEAK inhibitor, an IL-6 inhibitor, an IL-6 receptor inhibitor, a soluble lymphotoxin beta receptor, or a soluble BAFF antagonist. The NSAID can be a COX-2 inhibitor. The DMARD can be methotrexate, gold, penicillamine, or hydroxychloroquine.

Herein described is a composition containing at least two (e.g., at least three, at least four, at least five, at least six, at least seven, at least eight, at least nine, at least 10, at least 11, at least 12, at least 15, at least 20, at least 22, or at least 24 or more) polynucleotides that selectively hybridize to all of part of each of at least two (e.g., at least three, at least four, at least five, at least six, at least seven, at least eight, at least nine, at least 10, at least 11, at least 12, at least 15, at least 20, at least 22, or at least 24 or more, respectively) genes selected from the group consisting of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, or SFRS2. The at least five genes can include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6. The at least five genes can include, e.g., CLTB, COL4A3BP, CXorf52, FAM44A, MXRA7, PGK1, SFRS2, or YIPF6. The at least eight genes can include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and/or YIPF6. The at least eight genes can consist of, or include, e.g., CLTB, COL4A3BP, CXorf52, FAM44A, MXRA7, PGK1, SFRS2, and/or YIPF6. The at least 24 genes can consist of, or include, e.g., ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6.

In some of the compositions described above, the at least two polynucleotides can be bound to a solid support.

The solid support can be a microarray chip, a particle (e.g., an encoded, magnetic, or magnetic and encoded particle), or any other solid support described herein.

Any of the compositions described above can contain less than 100,000 (e.g., less than 90,000; less than 80,000; less than 70,000; less than 60,000; less than 50,000; less than 40,000; less than 30,000; less than 20,000; less than 15,000; less than 10,000; less than 5,000; less than 4,000; less than 3,000; less than 2,000; less than 1,500; less than 1,000; less than 750; less than 500, less than 200, less than 100, or less than 50) different polynucleotides.

The kit can include instructions for administering a therapy containing an anti-TNF agent if the expression level of one or more of ANKIB1, ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, or ZFP36L1 are determined to be elevated or the expression level of one or more of ANKRD12, CAMK2G-, CASP5, CXorf52, DNAH1, EEA1, FAM44A, FOXJ3, HDAC4, MNT, MXRA7, PTCH1, SEL1L, or SFRS2 are determined to be reduced.

Herein described is an anti-TNF therapy response profile for a subject obtained by a method that includes the steps of: providing a biological sample from a subject; measuring the expression level of one or more genes in the biological sample, wherein the one or more genes comprise at least ANKIB1 and may further comprise one gene selected from the group consisting of ARF1, ARF5, BRWD2, CALM2, CLTB, COL4A3BP, C9orf80, EGLN2, HDAC5, LGALS9, MYLIP, PCBP2, PGK1, RBBP4, RER1, RPA3, SERF2, SLC25A39, SRGAP2, TUG1, YIPF6, ZNF294, ZFP36L1, ANKRD12, CAMK2G-, CASP5, CXorf52, DNAH1, EEA1, FAM44A, FOXJ3, HDAC4, MNT, MXRA7, PTCH1, SEL1L, and SFRS2; and assessing the expression level of at least two of the one or more genes in the biological sample to obtain an anti-TNF response profile for the subject.

Any of the response profiles described above can be used for predicting the response of a subject to a therapy comprising an anti-TNF agent.

As used herein, an anti-TNF agent is one that inhibits the activity of TNF. Inhibition of the activity of TNF includes, for example, inhibition of the expression (mRNA or protein expression) of TNF, inhibition of the release of TNF from a cell in which it is produced, or inhibition of the ability of TNF to bind to and/or activate its cognate receptor. Agents that inhibit the activity of TNF include, but are not limited to, small molecules, small interfering RNAs (siRNAs), anti-sense RNAs, antibodies that specifically bind to TNF, soluble TNF receptors, or dominant negative-TNF molecules (such as a dominant negative TNF protein or a nucleic acid encoding a dominant negative TNF protein). It is understood that an agent that inhibits TNF can be one that inhibits the ability of TNF to activate a receptor, but does not inhibit the binding of TNF to the receptor. Anti-TNF antibodies include, e.g., infliximab (Remicade®), D2E7 (adalumimab; Humira™), certolizumab (CDP-870), and CDP-571 (see, e.g., Sandborn et al. (2004) Gut 53(10):1485-1493; Choy et al. (2002) Rheumatology 41(10):1133-1137; and Kaushik et al. (2005) Expert Opinion on Biological Therapy 5(4):601-606(6)). Soluble TNF receptors include, e.g., etanercept (sTNF-RII:Fc; Enbrel®). Exemplary anti-TNF therapies are described in, e.g., U.S. Patent No. 6,270,766.

A non-anti-TNF agent can be, e.g., a non-steroidal anti-inflammatory drug (NSAID), a corticosteroid (e.g., glucocorticoid or mineralocorticoids), or a disease-modifying antirheumatic drug (DMARD). NSAIDS include, e.g., COX-2 inhibitors (e.g., celecoxib, etoricoxib, or lumiracoxib), salicylates (e.g., aspirin, amoxiprin, benorilate, choline magnesium salicylate, diflunisal, faislamine, methyl salicylate, magnesium salicylate, or salicyl salicylate (salsalate)), arylalkanoic acids (e.g., diclofenac, aceclofenac, acemetacin, bromfenac, etodolac, indometacin, ketorolac, nabumetone, sulindac, or tolmetin), or pyrazolidine derivatives (such as phenylbutazone, azapropazone, metamizole, oxyphenbutazone, or sulfinprazone). DMARDS include, but are not limited to, adalimumab, azathioprine, anti-malarials (e.g., chloroquine or hydroxychloroquine), cyclosporine A, D-penicillamine, gold salts (e.g., sodium aurothiomalate or auranofin), leflunomide , methotrexate (MTX), minocycline, or sulfasalazine (SSZ). Non-anti-TNF therapies also include anti-CD20 antibodies (e.g., rituximab (Rituxan®)), TWEAK inhibitors (e.g., anti-TWEAK antibodies, see, e.g., WO 06/122187), soluble lymphotoxin beta receptors (e.g., LTBR-Fc), BAFF antagonists such as BR3-Fc; and IL-6 inhibitors (e.g., IL-6 antagonist antibodies, soluble IL-6 receptors) and IL-6 receptor inhibitors (e.g., IL-6 receptor antagonist antibodies such as tocilizumab or atlizumab (Actemra™) see, e.g., WO/2004/096273; EP1536012, WO/2006/119115, U.S. Patent Nos. 5,559,012 and 5,888,510; and U.S. Publication No. 20010001663). The non-anti-TNF agent can be an anti-inflammatory agent that does not contain an anti-TNF agent.

Diseases treatable by a therapy comprising an anti-TNF agent include, e.g., immune (e.g., inflammatory) disorders, infections, neurodegenerative diseases, malignant pathologies involving TNF-secreting tumors or other malignancies involving TNF, and alcohol-induced hepatitis.

Immune or inflammatory disorders include, but are not limited to, allergic bronchopulmonary aspergillosis; allergic rhinitis, autoimmune hemolytic anemia; acanthosis nigricans; allergic contact dermatitis; Addison's disease; atopic dermatitis; alopecia areata; alopecia universalis; amyloidosis; anaphylactoid purpura; anaphylactoid reaction; aplastic anemia; angioedema, hereditary; angioedema, idiopathic; ankylosing spondylitis; arteritis, cranial; arteritis, giant cell; arteritis, Takayasu's; arteritis, temporal; asthma; ataxia-telangiectasia; autoimmune oophoritis; autoimmune orchitis; autoimmune polyendocrine failure; Behcet's disease; Berger's disease; Buerger's disease; bronchitis; bullous pemphigus; candidiasis, chronic mucocutaneous; Caplan's syndrome; post-myocardial infarction syndrome; post-pericardiotomy syndrome; carditis; celiac sprue; Chagas's disease; Chediak-Higashi syndrome; Churg-Strauss disease; Cogan's syndrome; cold agglutinin disease; CREST syndrome; Crohn's disease; cryoglobulinemia; cryptogenic fibrosing alveolitis; dermatitis herpetifomis; dermatomyositis; diabetes mellitus; Diamond-Blackfan syndrome; DiGeorge syndrome; discoid lupus erythematosus; eosinophilic fasciitis; episcleritis; drythema elevatum diutinum; erythema marginatum; erythema multiforme; erythema nodosum; Familial Mediterranean fever; Felty's syndrome; pulmonary fibrosis; glomerulonephritis, anaphylactoid; glomerulonephritis, autoimmune; glomerulonephritis, post-streptococcal; glomerulonephritis, post-transplantation; glomerulopathy, membranous; Goodpasture's syndrome; granulocytopenia, immune-mediated; granuloma annulare; granulomatosis, allergic; granulomatous myositis; Grave's disease; Hashimoto's thyroiditis; hemolytic disease of the newborn; hemochromatosis, idiopathic; Henoch- Schoenlein purpura; hepatitis, chronic active and chronic progressive; histiocytosis X; hypereosinophilic syndrome; idiopathic thrombocytopenic purpura; Job's syndrome; juvenile dermatomyositis; juvenile rheumatoid arthritis (Juvenile chronic arthritis); Kawasaki's disease; keratitis; keratoconjunctivitis sicca; Landry-Guillain-Barre-Strohl syndrome; leprosy, lepromatous; Loeffler's syndrome; lupus; Lyell's syndrome; lyme disease; lymphomatoid granulomatosis; mastocytosis, systemic; mixed connective tissue disease; mononeuritis multiplex; Muckle-Wells syndrome; mucocutaneous lymph node syndrome; mucocutaneous lymph node syndrome; multicentric reticulohistiocytosis; multiple sclerosis; myasthenia gravis; mycosis fungoides; necrotizing vasculitis, systemic; nephrotic syndrome; overlap syndrome; panniculitis; paroxysmal cold hemoglobinuria; paroxysmal nocturnal hemoglobinuria; pemphigoid; pemphigus; pemphigus erythematosus; pemphigus foliaceus; pemphigus vulgaris; pigeon breeder's disease; pneumonitis, hypersensitivity; polyarteritis nodosa; polymyalgia rheumatic; polymyositis; polyneuritis, idiopathic; portuguese familiar polyneuropathies; pre-eclampsia/eclampsia; primary biliary cirrhosis; progressive systemic sclerosis (scleroderma); psoriasis; psoriatic arthritis; pulmonary alveolar proteinosis; pulmonary fibrosis, Raynaud's phenomenon/syndrome; Reidel's thyroiditis; Reiter's syndrome, relapsing polychrondritis; rheumatic fever; rheumatoid arthritis; sarcoidosis; scleritis; sclerosing cholangitis; serum sickness; Sezary syndrome; Sjogren's syndrome; Stevens- Johnson syndrome; Still's disease; subacute sclerosing panencephalitis; sympathetic ophthalmia; systemic lupus erythematosus; yransplant rejection; ulcerative colitis; undifferentiated connective tissue disease; urticaria, chronic; urticaria, cold; uveitis; vitiligo; Weber-Christian disease; Wegener's granulomatosis, or Wiskott-Aldrich syndrome.

Infections can include, e.g., sepsis syndrome, cachexia, circulatory collapse and shock resulting from acute or chronic bacterial infection, acute and chronic parasitic and/or infectious diseases, bacterial infections, viral infections, or fungal infections.

Neurodegenerative diseases include, e.g., demyelinating diseases (such as multiple sclerosis and acute transverse myelitis); extrapyramidal and cerebellar disorders' such as lesions of the corticospinal system; disorders of the basal ganglia or cerebellar disorders; hyperkinetic movement disorders such as Huntington's Chorea and senile chorea; drug-induced movement disorders, such as those induced by drugs which block CNS dopamine receptors; hypokinetic movement disorders, such as Parkinson's disease; progressive supranucleo palsy; cerebellar and spinocerebellar disorders, such as astructural lesions of the cerebellum; spinocerebellar degenerations (spinal ataxia, Friedreich's ataxia, cerebellar cortical degenerations, multiple systems degenerations (Mencel, Dejerine-Thomas, Shi-Drager, and Machado-Joseph); and systemic disorders (Refsum's disease, abetalipoprotemia, ataxia telangiectasia, and mitochondrial multisystem disorder); demyelinating core disorders, such as multiple sclerosis, acute transverse myelitis; disorders of the motor unit, such as neurogenic muscular atrophies (anterior horn cell degeneration, such as amyotrophic lateral sclerosis, infantile spinal muscular atrophy and juvenile spinal muscular atrophy); Alzheimer's disease; Down's Syndrome in middle age; Diffuse Lewy body disease; Senile Dementia of Lewy body type; Wemicke-Korsakoff syndrome; chronic alcoholism; Creutzfeldt-Jakob disease; subacute sclerosing panencephalitis; Hallerrorden-Spatz disease; and dementia pugilistica.

Malignant pathologies involving TNF-secreting tumors or other malignancies involving TNF include, but not limited to leukemias (acute, chronic myelocytic, chronic lymphocytic and/or myelodyspastic syndrome); or lymphomas (Hodgkin's and non-Hodgkin's lymphomas, such as malignant lymphomas (Burkitt's lymphoma or mycosis fungoides).

Additional disorders that can be treated according to the methods described herein are described in, e.g., WO 06/74399, WO 06/122187, or U.S. Patent Nos. 5,656,272 and 5,919,452.

Sequence "complementarity," as used herein, refers to the chemical affinity between specific nitrogenous bases as a result of their hydrogen bonding properties (i.e., the property of two nucleic acid chains having base sequences such that an antiparallel duplex can form where the adenines and uracils (or thymine, in the case of DNA or modified RNA) are apposed to each other, and the guanines and cytosines are apposed to each other). Fully complementary sequences, thus, would be two sequences that have complete one-to-one correspondence (i.e., adenine to uracil and guanine to cytosine) of the base sequences when the nucleotide sequences form an antiparallel duplex.

Unless otherwise defined, all technical and scientific terms used herein have the same meaning as commonly understood by one of ordinary skill in the art to which this invention belongs. The materials, methods, and examples are illustrative only and not intended to be limiting.

Other features and advantages of the invention will be apparent from the following detailed description, and from the claims.

## DETAILED DESCRIPTION

Provided herein are methods and compositions (e.g., nucleic acid arrays and kits) for predicting the response of a subject (such as a human patient) to an anti-TNF therapy. In addition, the disclosure provides predictive biomarkers (e.g., gene expression levels) to identify those subjects for whom administering an anti-TNF therapy is likely to be effective or ineffective. Such biomarkers, compositions, and methods are useful in selecting appropriate therapeutic modalities (e.g., an anti-TNF therapy or a non-anti-TNF therapy) for subjects suffering from diseases such as rheumatoid arthritis, Crohn's disease, psoriatic arthritis, Behçet's disease, ankylosing spondylitis, and other immune disorders.

Gene Expression and Responsiveness to Anti-TNF Therapy

A series of genes have been identified whose expression levels (e.g., mRNA or protein expression levels) are predictive of the responsiveness or non-responsiveness of a subject to an anti-TNF therapy. The genes (and their corresponding Entrez Gene ID Nos.) are depicted in Table 1.

Table 1.

| Gene Name | Entrez Gene ID No. | SEQ ID NO |
|---|---|---|
| ANKIB1 | 54467 | SEQ ID NO: 1 |
| ANKRD12 | 23253 | SEQ ID NO: 2 |
| ARF1 | 375 | SEQ ID NO: 3 |
| ARF5 | 381 | SEQ ID NO: 4 |
| BRWD2 | 55717 | SEQ ID NO: 5 |
| C9orf80 | 58493 | SEQ ID NO: 6 |
| CALM2 | 805 | SEQ ID NO: 7 |
| CAMK2G- | 818 | SEQ ID NO: 8 |
| CASP5 | 838 | SEQ ID NO: 9 |
| CLTB | 1212 | SEQ ID NO: 10 |
| COL4A3BP | 10087 | SEQ ID NO: 11 |
| CXorf52 | 286967 | SEQ ID NO: 12 |
| DNAH1 | 25981 | SEQ ID NO: 13 |
| EEA1 | 8411 | SEQ ID NO: 14 |
| EGLN2 | 112398 | SEQ ID NO: 15 |
| FAM44A | 259282 | SEQ ID NO: 16 |
| FOXJ3 | 22887 | SEQ ID NO: 17 |
| HDAC4 | 9759 | SEQ ID NO: 18 |
| HDAC5 | 10014 | SEQ ID NO: 19 |
| LGALS9 | 3965 | SEQ ID NO: 20 |
| MNT | 4335 | SEQ ID NO: 21 |
| MXRA7 | 439921 | SEQ ID NO: 22 |
| MYLIP | 29116 | SEQ ID NO: 23 |
| PCBP2 | 5094 | SEQ ID NO: 24 |
| PGK1 | 5230 | SEQ ID NO: 25 |
| PTCH1 | 5727 | SEQ ID NO: 26 |
| RBBP4 | 5928 | SEQ ID NO: 27 |
| RER1 | 11079 | SEQ ID NO: 28 |

(continued)

| Gene Name | Entrez Gene ID No. | SEQ ID NO |
|-----------|--------------------|-----------|
| RPA3 | 6119 | SEQ ID NO: 29 |
| SEL1L | 6400 | SEQ ID NO: 30 |
| SERF2 | 10169 | SEQ ID NO: 31 |
| SFRS2 | 6427 | SEQ ID NO: 32 |
| SLC25A39 | 51629 | SEQ ID NO: 33 |
| SRGAP2 | 23380 | SEQ ID NO: 34 |
| TUG1 | 65000 | SEQ ID NO: 35 |
| YIPF6 | 286451 | SEQ ID NO: 36 |
| ZFP36L1 | 677 | SEQ ID NO: 37 |
| ZNF294 | 26046 | SEQ ID NO: 38 |

An elevated expression level of a gene can be predictive of either responsiveness or non-responsiveness to an anti-TNF therapy. For example, an elevated expression level of one or more of HDAC4, CXorf52, ANKRD12, CAMK2G-, CASP5, MXRA7, FAM44A, MNT, SEL1L, EEA1, FOXJ3, DNAH1, PTCH1, or SFRS2 predicts that a subject will not respond to an anti-TNF therapy whereas an elevated expression level of one or more of CLTB, RBBP4, COL4A3BP, C9orf80, PCBP2, YIPF6, MYLIP, ZNF294, RER1, CALM2, ARF5, ARF1, HDAC5, ANKIB1, BRWD2, PGK1, ZFP36L1, SERF2, SRGAP2, TUG1, LGALS9, SLC25A39, EGLN2, or RPA3 predicts that a subject will respond to an anti-TNF therapy.

A decreased expression level of a gene can be predictive of either responsiveness or non-responsiveness to an anti-TNF therapy. For example, a decreased expression level of one or more of CLTB, RBBP4, COL4A3BP, C9orf80, PCBP2, YIPF6, MYLIP, ZNF294, RER1, CALM2, ARF5, ARF1, HDAC5, ANKIB1, BRWD2, PGK1, ZFP36L1, SERF2, SRGAP2, TUG1, LGALS9, SLC25A39, EGLN2, or RPA3 predicts that a subject will not respond to an anti-TNF therapy whereas a decreased expression level of one or more of HDAC4, CXorf52, ANKRD12, CAMK2G-, CASP5, MXRA7, FAM44A, MNT, SEL1L, EEA1, FOXJ3, DNAH1, PTCH1, or SFRS2 predicts that a subject will respond to an anti-TNF therapy.

In predicting responsiveness or non-responsiveness to an anti-TNF therapy, the expression level of one or more of the genes depicted in Table 1 can be elevated or reduced by at least about 1.5 fold (e.g., at least about 2 fold, at least about 2.5 fold, at least about 3.0 fold, at least about 3.5 fold, at least about 4.0 fold, or at least about 5 fold or more).

Gene expression can be detected as, e.g., protein or mRNA expression of a target gene. That is, the presence or expression level (amount) of a gene can be determined by detecting and/or measuring the level of mRNA or protein expression of the gene. In some embodiments, gene expression can be detected as the activity of a protein encoded by a gene such as a gene depicted in Table 1.

A variety of suitable methods can be employed to detect and/or measure the level of mRNA expression of a gene. For example, mRNA expression can be determined using Northern blot or dot blot analysis, reverse transcriptase-PCR (RT-PCR; e.g., quantitative RT-PCR), *in situ* hybridization (e.g., quantitative *in situ* hybridization) or nucleic acid array (e.g., oligonucleotide arrays or gene chips) analysis. Details of such methods are described below and in, e.g., Sambrook et al., Molecular Cloning: A Laboratory Manual Second Edition vol. 1, 2 and 3. Cold Spring Harbor Laboratory Press: Cold Spring Harbor, New York, USA, Nov. 1989; Gibson et al. (1999) Genome Res. 6(10):995-1001; and Zhang et al. (2005) Environ. Sci. Technol. 39(8):2777-2785; U.S. Publication No. 2004086915; European Patent No. 0543942; and U.S. Patent No. 7,101,663.

In one example, the presence or amount of one or more discrete mRNA populations in a biological sample can be determined by isolating total mRNA from the biological sample (see, e.g., Sambrook et al. (*supra*) and U.S. Patent No. 6,812,341) and subjecting the isolated mRNA to agarose gel electrophoresis to separate the mRNA by size. The size-separated mRNAs are then transferred (e.g., by diffusion) to a solid support such as a nitrocellulose membrane. The presence or amount of one or more mRNA populations in the biological sample can then be determined using one or more detectably-labeled-polynucleotide probes, complementary to the mRNA sequence of interest, which bind to and thus render detectable their corresponding mRNA populations. Detectable-labels include, e.g., fluorescent (e.g., umbelliferone, fluorescein, fluorescein isothiocyanate, rhodamine, dichlorotriazinylamine fluorescein, dansyl chloride, allophycocyanin (APC), or phycoerythrin), luminescent (e.g., europium, terbium, Qdot™ nanoparticles supplied by the Quantum Dot Corporation, Palo Alto, CA), radiological (e.g., $^{125}$I, $^{131}$I, $^{35}$S, $^{32}$P, $^{33}$P, or $^{3}$H), and enzymatic (horseradish peroxi-

dase, alkaline phosphatase, beta-galactosidase, or acetylcholinesterase) labels.

In another example, the presence or amount of discrete populations of mRNA (e.g., mRNA encoded by one or more genes depicted in Table 1) in a biological sample can be determined using nucleic acid (or oligonucleotide) arrays (e.g., an array described below under "Arrays and Kits"). For example, isolated mRNA from a biological sample can be amplified using RT-PCR with random hexamer or oligo(dT)-primer mediated first strand synthesis. The RT-PCR step can be used to detectably-label the amplicons, or, optionally, the amplicons can be detectably-labeled subsequent to the RT-PCR step. For example, the detectable-label can be enzymatically (e.g., by nick-translation or kinase such as T4 polynucleotide kinase) or chemically conjugated to the amplicons using any of a variety of suitable techniques (see, e.g., Sambrook et al., *supra*). The detectably-labeled-amplicons are then contacted to a plurality of polynucleotide probe sets, each set containing one or more of a polynucleotide (e.g., an oligonucleotide) probe specific for (and capable of binding to) a corresponding amplicon, and where the plurality contains many probe sets each corresponding to a different amplicon. Generally, the probe sets are bound to a solid support and the position of each probe set is predetermined on the solid support. The binding of a detectably-labeled amplicon to a corresponding probe of a probe set indicates the presence or amount of a target mRNA in the biological sample. Additional methods for detecting mRNA expression using nucleic acid arrays are described in, e.g., U.S. Patent Nos. 5,445,934; 6,027,880; 6,057,100; 6,156,501; 6,261,776; and 6,576,424.

Methods of detecting and/or for quantifying a detectable label depend on the nature of the label. The products of reactions catalyzed by appropriate enzymes (where the detectable label is an enzyme; see above) can be, without limitation, fluorescent, luminescent, or radioactive or they may absorb visible or ultraviolet light. Examples of detectors suitable for detecting such detectable labels include, without limitation, x-ray film, radioactivity counters, scintillation counters, spectrophotometers, colorimeters, fluorometers, luminometers, and densitometers.

The expression of a gene can also be determined by detecting and/or measuring expression of a protein encoded by the gene. Methods of determining protein expression generally involve the use of antibodies specific for the target protein of interest. For example, methods of determining protein expression include, but are not limited to, western blot or dot blot analysis, immunohistochemistry (e.g., quantitative immunohistochemistry), immunocytochemistry, enzyme-linked immunosorbent assay (ELISA), enzyme-linked immunosorbent spot (ELISPOT; Coligan, J. E., et al., eds. (1995) Current Protocols in Immunology. Wiley, New York), or antibody array analysis (see, e.g., U.S. Publication Nos. 20030013208 and 2004171068). Further description of many of the methods above and additional methods for detecting protein expression can be found in, e.g., Sambrook et al. (*supra*).

In one example, the presence or amount of protein expression of a gene (e.g., a gene depicted in Table 1) can be determined using a western blotting technique. For example, a lysate can be prepared from a biological sample, or the biological sample itself, can be contacted with Laemmli buffer and subjected to sodium-dodecyl sulfate polyacrylamide gel electrophoresis (SDS-PAGE). SDS-PAGE-resolved proteins, separated by size, can then be transferred to a filter membrane (e.g., nitrocellulose) and subjected to immunoblotting techniques using a detectably-labeled antibody specific to the protein of interest. The presence or amount of bound detectably-labeled antibody indicates the presence or amount of protein in the biological sample.

In another example, an immunoassay can be used for detecting and/or measuring the protein expression of a gene (e.g., a gene depicted in Table 1). As above, for the purposes of detection, an immunoassay can be performed with an antibody that bears a detection moiety (e.g., a fluorescent agent or enzyme). Proteins from a biological sample can be conjugated directly to a solid-phase matrix (e.g., a multi-well assay plate, nitrocellulose, agarose, sepharose, encoded particles, or magnetic beads) or it can be conjugated to a first member of a specific binding pair (e.g., biotin or streptavidin) that attaches to a solid-phase matrix upon binding to a second member of the specific binding pair (e.g., streptavidin or biotin). Such attachment to a solid-phase matrix allows the proteins to be purified away from other interfering or irrelevant components of the biological sample prior to contact with the detection antibody and also allows for subsequent washing of unbound antibody. Here as above, the presence or amount of bound detectably-labeled antibody indicates the presence or amount of protein in the biological sample.

Methods for generating antibodies or antibody fragments specific for a protein encoded by one or more genes can be generated by immunization, e.g., using an animal, or by *in vitro* methods such as phage display. A polypeptide that includes all or part of a target protein can be used to generate an antibody or antibody fragment. The antibody can be a monoclonal antibody or a preparation of polyclonal antibodies.

Methods for detecting or measuring gene expression (e.g., mRNA or protein expression) can optionally be performed in formats that allow for rapid preparation, processing, and analysis of multiple samples. This can be, for example, in multi-welled assay plates (e.g., 96 wells or 386 wells) or arrays (e.g., nucleic acid chips or protein chips). Stock solutions for various reagents can be provided manually or robotically, and subsequent sample preparation (e.g., RT-PCR, labeling, or cell fixation), pipetting, diluting, mixing, distribution, washing, incubating (e.g., hybridization), sample readout, data collection (optical data) and/or analysis (computer aided image analysis) can be done robotically using commercially available analysis software, robotics, and detection instrumentation capable of detecting the signal generated from the assay. Examples of such detectors include, but are not limited to, spectrophotometers, luminometers, fluorimeters, and devices that measure radioisotope decay. Exemplary high-throughput cell-based assays (e.g., detecting the presence or level of a target protein in a cell) can utilize ArrayScan® VTI HCS Reader or KineticScan® HCS Reader technology (Cellomics Inc., Pittsburg, PA).

In some embodiments, the expression level (or activity) of at least two genes (e.g., at least three genes, at least four genes, at least five genes, at least six genes, at least seven genes, at least eight genes, at least nine genes, at least 10 genes, at least 11 genes, at least 12 genes, at least 13 genes, at least 14 genes, at least 15 genes, at least 16 genes, at least 17 genes, at least 18 genes, at least 19 genes, at least 20 genes, at least 21 genes, at least 22 genes, at least 23 genes, or at least 24 genes or more) can be assessed and/or measured.

To aid in detecting the presence or level of expression of one or more of the genes depicted in Table 1, the nucleic acid sequences of the genes can be used, e.g., as hybridization polynucleotide probes or primers (e.g., for amplification or reverse transcription). The probes and primers can be oligonucleotides of sufficient length to provide specific hybridization to a RNA or DNA target derived from a biological sample. Depending on the specific application, varying hybridization conditions can be employed to achieve varying degrees of selectivity of a probe or primer towards target sequence.

Standard stringency conditions are described by Sambrook, et al. (*supra*) and Haymes, et al. Nucleic Acid Hybridization, A Practical Approach, IRL Press, Washington, D.C. (1985). In order for a nucleic acid molecule to serve as a primer or probe it need only be sufficiently complementary in sequence to be able to form a stable double-stranded structure under the particular hybridization conditions (e.g., solvent and salt concentrations) employed.

Appropriate stringency conditions that promote DNA hybridization, for example, $6.0 \times$ sodium chloride/sodium citrate (SSC) at about 45° C, followed by a wash of $2.0 \times$ SSC at 50° C, are known to those skilled in the art or can be found in Ausubel, et al., Current Protocols in Molecular Biology, John Wiley & Sons, N.Y. (1989). For example, the salt concentration in the wash step can be selected from a low stringency of about $2.0 \times$ SSC at 50°C to a high stringency of about $0.2 \times$ SSC at 50°C. The temperature used in the wash step can be increased from low stringency conditions at room temperature (about 22° C) to high stringency conditions at about 65°C. Temperature and salt conditions may be varied independently.

Primers and probes can be used in hybridization assays or techniques or in a variety of PCR-type methods. The primers and probes can be detectably-labeled with reagents that facilitate detection (e.g., fluorescent labels, chemical labels (see, e.g., U.S. Patent Nos. 4,582,789 and 4,563,417), or modified bases).

In addition, the nucleic acid sequences (e.g., oligonucleotide probes) can be used in nucleic acid arrays (such as the nucleic acid arrays described below under "Arrays and Kits") for detection and/or quantitation of gene expression.

<u>Samples and Sample Collection</u>

Suitable biological samples for the methods described herein include any biological fluid, cell, tissue, or fraction thereof, which includes analyte biomolecules of interest such as nucleic acid (e.g., DNA or mRNA) or protein. A biological sample can be, for example, a specimen obtained from a subject (e.g., a mammal such as a human) or can be derived from such a subject. For example, a sample can be a tissue section obtained by biopsy, or cells that are placed in or adapted to tissue culture. A biological sample can also be a biological fluid such as urine, blood, plasma, serum, saliva, semen, sputum, cerebral spinal fluid, tears, or mucus, or such a sample absorbed onto a paper or polymer substrate. A biological sample can be further fractionated, if desired, to a fraction containing particular cell types. For example, a blood sample can be fractionated into serum or into fractions containing particular types of blood cells such as red blood cells or white blood cells (leukocytes). If desired, a sample can be a combination of samples from a subject such as a combination of a tissue and fluid sample.

The biological samples can be obtained from a subject, e.g., a subject having, suspected of having, or at risk of developing, an inflammatory disease such as rheumatoid arthritis or Crohn's disease. Any suitable methods for obtaining the biological samples can be employed, although exemplary methods include, e.g., phlebotomy, swab (e.g., buccal swab), or fine needle aspirate biopsy procedure. Non-limiting examples of tissues susceptible to fine needle aspiration include lymph node, lung, thyroid, breast, and liver. Samples can also be collected, e.g., by microdissection (e.g., laser capture microdissection (LCM) or laser microdissection (LMD)), bladder wash, smear (PAP smear), or ductal lavage.

Methods for obtaining and/or storing samples that preserve the activity or integrity of molecules (e.g., nucleic acids or proteins) in the sample are well known to those skilled in the art. For example, a biological sample can be further contacted with one or more additional agents such as appropriate buffers and/or inhibitors, including nuclease, protease and phosphatase inhibitors, which preserve or minimize changes in the molecules (e.g., nucleic acids or proteins) in the sample. Such inhibitors include, for example, chelators such as ethylenediamine tetraacetic acid (EDTA), ethylene glycol bis(P-aminoethyl ether) N,N,N1,N1-tetraacetic acid (EGTA), protease inhibitors such as phenylmethylsulfonyl fluoride (PMSF), aprotinin, leupeptin, antipain and the like, and phosphatase inhibitors such as phosphate, sodium fluoride, vanadate and the like. Appropriate buffers and conditions for isolating molecules are well known to those skilled in the art and can be varied depending, for example, on the type of molecule in the sample to be characterized (see, for example, Ausubel et al. Current Protocols in Molecular Biology (Supplement 47), John Wiley & Sons, New York (1999); Harlow and Lane, Antibodies: A Laboratory Manual (Cold Spring Harbor Laboratory Press (1988); Harlow and Lane, Using Antibodies: A Laboratory Manual, Cold Spring Harbor Press (1999); Tietz Textbook of Clinical Chemistry, 3rd ed. Burtis and Ashwood, eds. W.B. Saunders, Philadelphia, (1999)). A sample also can be processed to eliminate or minimize the presence of interfering substances. For example, a biological sample can be fractionated or purified to remove one or more materials that are not of interest. Methods of fractionating or purifying a biological sample include, but are not limited to, chromatographic methods such as liquid chromatography, ionexchange chromatography, size-exclusion chromatography, or affinity chromatography.

For use in the methods described herein, a sample can be in a variety of physical states. For example, a sample can be a liquid or solid, can be dissolved or suspended in a liquid, can be in an emulsion or gel, and can be absorbed onto a material.

Exemplary biological samples, methods for obtaining the samples, and purification methods (e.g., mRNA purification methods) are detailed in the accompanying Examples.

Applications

The methods and compositions described herein can be used to, e.g., (a) predict the responsiveness or non-responsiveness of a subject (e.g., a human) to an anti-TNF therapy and/or (b) generate an anti-TNF therapy response profile for a subject. The profile can include information that indicates whether one or more genes, such as one or more genes depicted in Table 1, are expressed (e.g., yes or no) and/or information that indicates the expression level of one or more genes (e.g., one or more genes depicted in Table 1).

The response profiles described herein can contain information on the expression or expression level of at least two or more (e.g., at least three or more, at least four or more, at least five or more, at least six or more, at least seven or more, at least eight or more, at least nine or more, at least 10 or more, at least 11 or more, at least 12 or more, at least 13 or more, at least 14 or more, at least 15 or more, at least 16 or more, at least 17 or more, at least 18 or more, at least 19 or more, at least 20 or more, at least 21 or more, at least 22 or more, at least 23 or more, or at least 24 or more) genes depicted in Table 1.

Grouping of multiple genes (e.g., 2, 3, 4, 5, 6, 7, 8, 9, 10, 11, 12, 13, 14, 15, 16, 17, 18, 19, 20, 21, 22, 23, or 24 or more genes depicted in Table 1) into sets or clusters can improve the sensitivity or specificity of the classifiers for response or non-response of a subject to an anti-TNF therapy. For example, the accompanying Examples describe the grouping of the genes depicted in Table 1 into 8 clusters. A group of genes comprising individual genes selected from each of the clusters can then be tested for predictive accuracy and the classifiers can be recalculated based on the group of genes. This can result in, e.g., a classifier (e.g., an 8 gene or a 24 gene classifier) with an improved accuracy of prediction as compared to a single gene-based classifier. Any grouping of genes from each cluster can lead to a more accurate classifier. Genes within each cluster can in many cases be substituted for one another without significantly compromising accuracy. Furthermore, any gene depicted in Table 1 could be added back to the grouping (e.g., the grouping of 8 genes or 24 genes).

It is understood that some genes (e.g., some of the genes depicted in Table 1) can be expressed in a cell- or tissue-specific manner. However, such differences in expression can be exploited in several ways. For example, in embodiments where a particular cell type (e.g. neutrophils, monocyte, or B cells) is being analyzed, it can be useful to select one or more gene (or a group of genes) that are expressed in that cell type. Such a strategy can also be useful, e.g., where a biological sample contains multiple cell or tissue types, but only a particular cell or tissue type is to be analyzed. Thus, selecting a particular set of genes expressed only, or predominantly, in the target cell type of interest can provide more a focused prediction.

The resultant information (anti-TNF therapy response profile) can be used for predicting the response of a subject (e.g., a human patient) to an anti-TNF therapy, such as any of the anti-TNF therapies described herein. In addition, the response profiles can be used in predicting the response of a subject to a variety of therapies and/or a variety of disease states since, e.g., the expression levels of one or more of the genes (e.g., one or more of the genes depicted in Table 1) examined can be indicative of such responses or disorders, whether or not physiologic or behavioral symptoms of the disorder have become apparent.

Responsiveness (and, conversely, non-responsiveness) of a subject to a therapy comprising an anti-TNF agent can be classified in several ways and classification is dependent on the subject's disease (e.g., rheumatoid arthritis, Crohn's disease, or any other of the diseases treatable by therapy comprising an anti-TNF agent), the severity of the disease, and the particular medicament the subject is administered. For example, responsiveness of a subject (e.g., a human) with rheumatoid arthritis can be classified as achieving at least about 20% (e.g., at least about 25%, at least about 30%, at least about 35%, at least about 40%, at least about 45%, at least about 50%, at least least 55%, at least about 60%, at least about 65%, or at least about 70% or more) improvement in one or more (e.g., two or more, three or more, four or more, five or more, or more than six) of a number of objective clinical indicia (e.g., American College of Rheumatology (ACR)-core set measures) such as, but not limited to, tender joint count, swollen joint count, pain, global self-assessment of improvement by a subject, global assessment of improvement of the subject by a physician, improvement of a subject's disability, or presence or amount of an acute-phase reactant (e.g., as determined by erythrocyte sedimentation rate (ESR) or presence or level of C-reactive protein). That is, a subject can be classified as responsive to an anti-TNF therapy if, e.g., he or she exhibits about 20% improvement in tender and swollen joint counts and 20% improvement in 3 of the 5 remaining ACR-core set measures (see above). In some instances, a subject is classified as responsive to an anti-TNF therapy using an ACR criteria of 20% (ACR20%), 50% (ACR50%), or 70% (ACR70%). Such improvements can be compared to, e.g., the responsiveness of the same subject to a placebo.

The responsiveness of a subject to an anti-TNF therapy can also be classified using a Disease Activity Score (DAS or DAS28), which is a measure of the number of swollen and tender joints and the ESR or CRP. For example, the DAS28 can be measured by analyzing the number of swollen joints, the number of tender joints, the ESR (in mm/hr) and the VAS (visual analog scale) general health of the patient. Under DAS criteria, responsiveness of a subject to an anti-TNF therapy is a DAS improvement of at least about 1.2 (e.g., at least about 1.3, at least about 1.4, at least about 1.5, at least about 1.6. at least about 1.7, at least about 1.8, at least about 1.9, at least about 2.0, at least about 2.1, at least about 2.2, at least about 2.3, at least about 2.4, at least about 2.5 or at least about 2.6 or more), e.g., at least about 1 month (e.g., at least about 1.5 months, at least about 2.0 months, at least about 2.5 months, at least about 3.0 months, at least about 4.0 months, at least about 5 months, or at least about 6 months). The results of the DAS and the DAS28 are not directly interchangeable as the DAS has a range varying from 1 to 9 and the DAS28 has a range of from 2 to 10; however, a transformation formula can be used to calculate the DAS28 from the DAS: DAS28= (1,072 x DAS) + 0,938.

Additional information regarding the DAS (e.g., methods to calculate the DAS) and ACR classifications of responsiveness of a subject to an anti-TNF therapy can be found in, e.g., Braun et al. (2003) Ann. Rheum. Dis. 62: 1023-1024; Felson et al. (1995) Arthritis and Rheumatism 38(6); Verhoeven et al. (2000) 59:966-974; at the American College of Rheumatology website; or at the website for the Department of Rheumatology, University Medical Centre Nijmegen, Netherlands.

Subjects of all ages can be affected by disorders treatable by an anti-TNF therapy. For example, the first symptoms of Crohn's disease can manifest in early adolescence to mid-life. Therefore, a biological sample used in a methods described herein can be obtained from a subject (e.g., a human) of any age, including a child, an adolescent, or an adult, such as an adult having, or suspected of having, a disease treatable by an anti-TNF therapy (e.g., rheumatoid arthritis).

The methods can also be applied to individuals at risk of developing a disorder treatable by an anti-TNF therapy. Such individuals include those who have (i) a family history of (a genetic predisposition for) such disorders or (ii) one or more risk factors for developing such disorders.

The methods described herein can involve, e.g., assessing the expression level (e.g., mRNA or protein expression level) of one or more genes (e.g., one or more genes depicted in Table 1), wherein the expression level of one or more of the genes predicts the response of a subject to an anti-TNF therapy. "Assessing" can include, e.g., comparing the expression of one or more genes in a test biological sample with a known or a control expression level (e.g., in a reference biological sample) of the particular gene(s) of interest. For example, the expression level of one or more genes in a test biological sample can be compared to the corresponding expression levels in a healthy subject, or an average expression level of multiple (e.g., two, three, four, five, six, seven, eight, nine, 10, 15, 20, 25, 30, 35, or 40 or more) healthy subjects, of the same species. The control sample can also be the expression level (or average expression level) of one or more subjects who have either responded to an anti-TNF therapy or not responded to an anti-TNF therapy. Assessing can also include determining if the expression level of one or more genes (e.g., one or more genes as depicted in Table 1) falls within a range of values predetermined as predictive of responsiveness or non-responsiveness of a subject to an anti-TNF therapy. In some embodiments, assessing can be, or include, determining if the expression of one or more genes (e.g., one or more of the genes depicted in Table 1) falls above or below a predetermined cut-off value. A cut-off value is typically an expression level of a gene, or ratio of the expression level of a gene with the expression level of another gene, above or below which is considered predictive of responsiveness or non-responsiveness of a subject to an anti-TNF therapy or, e.g., cause for retest. Thus, in accordance with the methods (and compositions) described herein, a reference expression level of a gene (e.g., a gene depicted in Table 1) is identified as a cut-off value, above or below of which is predictive of responsiveness or non-responsiveness to an anti-TNF therapy. It is understood that an anti-TNF therapy response profile can be interpreted as a whole (the expression level of all genes in the profile), in parts (certain collections or groups of genes (e.g., 8 or 24 genes) within the profile) or on a gene-by-gene basis.

Some cut-off values are not absolute in that clinical correlations can still remain significant over a range of values on either side of the cutoff; however, it is possible to select an optimal cut-off value (e.g. varying H-scores) of expression levels of genes for a particular sample types. Cut-off values determined for use in the methods described herein can be compared with, e.g., published ranges of expression levels but can be individualized to the methodology used and patient population. It is understood that improvements in optimal cut-off values could be determined depending on the sophistication of statistical methods used and on the number and source of samples used to determine reference level values for the different genes and sample types. Therefore, established cut-off values can be adjusted up or down, on the basis of periodic re-evaluations or changes in methodology or population distribution.

The reference expression level of one or more genes can be determined by a variety of methods. The reference level can be determined by comparison of the expression level of a gene of interest in, e.g., populations of subjects (e.g., patients) that are healthy, responsive to an anti-TNF therapy, or not responsive to an anti-TNF therapy. This can be accomplished, for example, by histogram analysis, in which an entire cohort of patients are graphically presented, wherein a first axis represents the expression level of a gene and a second axis represents the number of subjects in the cohort whose sample contain one or more expression levels at a given amount. Determination of the reference expression level of a gene can then be made based on an amount which best distinguishes these separate groups. The reference level can be a single number, equally applicable to every subject, or the reference level can vary, according to specific subpopulations of subjects. For example, older subjects can have a different reference level than younger subjects for the same metabolic disorder. In addition, a subject with more advanced disease (e.g., a more advanced form of a disease treatable by an anti-TNF therapy) can have a different reference value than one with a milder form of the disease.

Methods for determining reference expression levels of one or more genes are detailed in the accompanying Examples.

After predicting that a subject will respond or will not respond to an anti-TNF therapy, a medical practitioner (e.g., a doctor) can select the appropriate therapeutic modality for the subject (e.g., an anti-TNF therapy or a non-anti-TNF therapy, respectively). Selecting a therapy for a subject can be, e.g.: (i) writing a prescription for a medicament; (ii) giving (but not necessarily administering) a medicament to a subject (e.g., handing a sample of a prescription medication to a patient while the patient is at the physician's office); (iii) communication (verbal, written (other than a prescription), or electronic (email, post to a secure site)) to the patient of the suggested or recommended therapeutic modality (e.g., an anti-TNF therapy or a non-anti-TNF therapy); or (iv) identifying a suitable therapeutic modality for a subject and disseminating the information to other medical personnel, e.g., by way of patient record. The latter (iv) can be useful in a case where, e.g., more than one therapeutic agent are to be administered to a patient by different medical practitioners.

It is understood that an anti-TNF response profile can be in electronic form (e.g., an electronic patient record stored on a computer or other electronic (computer-readable) media such as a DVD, CD, or floppy disk) or written form. The anti-TNF response profile can also include information for several (e.g., two, three, four, five, 10, 20, 30, 50, or 100 or more) subjects (e.g., human patients). Such multi-subject response profiles can be used, e.g., in analyses (e.g., statistical analyses) of particular characteristics of subject cohorts.

After predicting that a subject will respond or will not respond to an anti-TNF therapy, a medical practitioner (e.g., a doctor) can administer the appropriate therapeutic modality to the subject (e.g., an anti-TNF therapy or a non-anti-TNF therapy, respectively). Methods of administering anti-TNF therapies such as an anti-TNF antibody or soluble TNF receptor are known in the art and described in, e.g., U.S. Patent Nos. 5,656,272; 6,193,969; 5,919,452. Likewise, methods for administering non-anti-TNF therapies such as a soluble lymphotoxin beta receptor or anti-CD20 antibody are known in the art and described in, for example, U.S. Patent Nos. 6,403,087; 5,925,351; 6,896,885; and 7,060,667.

It is understood that any therapy described herein (e.g., a therapy comprising an anti-TNF agent or a therapy that does not comprise an anti-TNF therapy) can include one or more additional therapeutic agents. That is, any therapy described herein can be co-administered (administered in combination) with one or more additional therapeutic agents such as, but not limited to, one or more of the non-anti-TNF agents described herein. For example, a therapy comprising an anti-TNF agent can include more than one anti-TNF agent (e.g., a soluble TNF receptor and an anti-TNF antibody). A therapy comprising an anti-TNF agent can also include, e.g., one or more non-anti-TNF agents such as methotrexate (MTX). Likewise, a non-anti-TNF therapy can include two or more non-anti-TNF agents, e.g., a TWEAK inhibitor and an IL-6 inhibitor or an IL-6 inhibitor and MTX. Furthermore, any therapy described herein can include one or more agents for treating, for example, pain, nausea, and/or one or more side-effects of a therapy comprising an anti-TNF agent or a non-anti-TNF therapy.

Combination therapies (e.g., co-administration of a therapy comprising an anti-TNF agent or non-anti-TNF therapy and one or more additional therapeutic agents) can be, e.g., simultaneous or successive. For example, an anti-TNF agent and one or more additional therapeutic agents can be administered at the same time or an anti-TNF agent can be administered first in time and the one or more additional therapeutic agents administered second in time. In some embodiments, the one or more additional therapeutic agents can be administered first in time and the anti-TNF agent administered second in time.

In cases where the subject predicted to respond to an anti-TNF therapy has been prior administered one or more non-anti-TNF therapies, the therapy comprising an anti-TNF agent can replace or augment a previously or currently administered therapy. For example, upon treating with the therapy comprising an anti-TNF agent, administration of the one non-anti-TNF therapies can cease or diminish, e.g., be administered at lower levels. Administration of the previous therapy can be maintained while the therapy comprising the anti-TNF agent is administered. In some embodiments, a previous therapy can be maintained until the level of the therapy comprising an anti-TNF agent reaches a level sufficient to provide a therapeutic effect.

In other embodiments, the subject can be monitored for a first pre-selected result, e.g., an improvement in one or more symptoms of a disease treatable by a therapy comprising an anti-TNF agent, e.g., rheumatoid arthritis or any other diseases treatable by therapy comprising an anti-TNF agent described herein. In some embodiments, when the first pre-selected result is observed, treatment with the therapy comprising an anti-TNF agent can be decreased or halted. In some embodiments, the subject can then be monitored for a second pre-selected result after treatment with the therapy comprising an anti-TNF agent is halted, e.g., a worsening (e.g., a worsening of a symptom) of a disease treatable by an anti-TNF agent. When the second pre-selected result is observed, administration of the therapy comprising an anti-TNF agent to the subject can be reinstated or increased, or administration of the first therapy can be reinstated, or the subject can be administered both a therapy comprising an anti-TNF agent, or an increased amount of a therapy comprising an anti-TNF agent, and the first therapeutic regimen.

Arrays and Kits

The kits and compositions are useful for predicting the response of a subject to an anti-TNF therapy.

The nucleic acid arrays can include at least two (e.g., at least three, at least four, at least five, at least six, at least seven, at least eight, at least nine, at least 10, at least 11, at least 12, at least 15, at least 20, at least 22, or at least 24) polynucleotides that hybridize to each of at least two (e.g., at least three, at least four, at least five, at least six, at least seven, at least eight, at least nine, at least 10, at least 11, at least 12, at least 15, at least 20, at least 22, or at least 24, respectively) genes depicted in Table 1. A polynucleotide can include coding sequence or noncoding sequence (e.g., exons, introns, or 5' or 3' regulatory sequences), e.g., of a gene depicted in Table 1. The polynucleotide can include sequence of the sense strand or the anti-sense strand of the coding sequence of a gene depicted in Table 1. The polynucleotide can also be single or double-stranded and of variable length. In some embodiments, the length of one strand of a polynucleotide that hybridizes to a gene (e.g., depicted in Table 1) can be about six nucleotides (e.g., about seven nucleotides, about eight nucleotides, about nine nucleotides, about 10 nucleotides, about 12 nucleotides, about 13 nucleotides, about 14 nucleotides, about 15 nucleotides, about 20 nucleotides, about 25 nucleotides, about 30 nucleotides, about 35 nucleotides, about 40 nucleotides, about 50 nucleotides, about 75 nucleotides, about 100 nucleotides, or about 150 or more nucleotides) in length. A longer polynucleotide often allows for higher stringency hybridization and wash conditions. The polynucleotide can be DNA, RNA, modified DNA or RNA, or a hybrid, where the nucleic acid contains any combination of deoxyribo- and ribo-nucleotides, and any combination of uracil, adenine, thymine, cytosine and guanine, as well as other bases such as inosine, xanthine, and hypoxanthine.

The polynucleotide arrays can be attached to a solid support, e.g., a porous or non-porous material that is insoluble. The substrate can be associated with the support in variety of ways, e.g., covalently or non-covalently bound.

A support can be composed of a natural or synthetic material, an organic or inorganic material. The composition of the solid support on which the polynucleotide sequences are attached (either 5' or 3' terminal attachment) generally depend on the method of attachment (e.g., covalent attachment). Suitable solid supports include, but are not limited to, plastics, resins, polysaccharides, silica or silica-based materials, functionalized glass, modified silicon, carbon, metals, inorganic glasses, membranes, nylon, natural fibers such as silk, wool and cotton, or polymers. The material comprising the solid support can have reactive groups such as carboxy, amino, or hydroxyl groups, which are used for attachment of the polynucleotides. Polymeric solid supports can include, e.g., polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly) vinylidenefluoride, polycarbonate, or polymethylpentene (see, e.g., U.S. Patent No. 5,427,779). Alternatively, the polynucleotide sequences can be attached to the solid support without the use of such functional groups.

Each polynucleotide (of a plurality of polynucleotides) on an array can be immobilized at predetermined positions such that each polynucleotide can be identified by its position. Exemplary polynucleotide arrays for use in the methods and kits described herein are described in, e.g., U.S. Patent Nos. 6,197,599; 5,902,723; and 5,871,928).

In some embodiments of any of the arrays described herein, the array of polynucleotides can have less than 100,000 (e.g., less than 90,000; less than 80,000; less than 70,000; less than 60,000; less than 50,000; less than 40,000; less than 30,000; less than 20,000; less than 15,000; less than 10,000; less than 5,000; less than 4,000; less than 3,000; less than 2,000; less than 1,500; less than 1,000; less than 750; less than 500, less than 200, less than 100, or less than 50) different polynucleotides.

The polynucleotide arrays can also be conjugated to solid support particles. Many suitable solid support particles are known in the art and illustratively include, e.g., particles, such as Luminex®-type encoded particles, magnetic particles, and glass particles.

Exemplary particles that can be used can have a variety of sizes and physical properties. Particles can be selected to have a variety of properties useful for particular experimental formats. For example, particles can be selected that remain suspended in a solution of desired viscosity or to readily precipitate in a solution of desired viscosity. Particles can be selected for ease of separation from sample constituents, for example, by including purification tags for separation with a suitable tag-binding material, paramagnetic properties for magnetic separation, and the like.

In some embodiments, encoded particles are used. Each particle includes a unique code (such as a bar code, luminescence code, fluorescence code, a nucleic acid code, and the like). Encoding can be used to provide particles for evaluating different nucleic acids in a single biological sample. The code is embedded (for example, within the interior of the particle) or otherwise attached to the particle in a manner that is stable through hybridization and analysis. The code can be provided by any detectable means, such as by holographic encoding, by a fluorescence property, color, shape, size, weight, light emission, quantum dot emission and the like to identify particle and thus the capture probes immobilized thereto. Encoding can also be the ratio of two or more dyes in one particle that is different than the ratio present in another particle. For example, the particles may be encoded using optical, chemical, physical, or electronic tags. Examples of such coding technologies are optical bar codes fluorescent dyes, or other means. In some embodiments, the particle code is a nucleic acid, e.g., a single stranded nucleic acid.

Different encoded particles can be used to detect or measure multiple nucleic acids (e.g., mRNAs) in parallel, so long as the encoding can be used to identify the polynucleotide (corresponding to an analyte nucleic acid) on a particular particle, and hence the presence or amount of the analyte nucleic acid (e.g., mRNA from a biological sample) being evaluated. A sample can be contacted with a plurality of such coded particles. When the particles are evaluated, e.g., using a fluorescent scanner, the particle code is read as is the fluorescence associated with the particle from any probe used to evaluate modification of the intact substrate associated with the particles.

One exemplary platform utilizes mixtures of fluorescent dyes impregnated into polymer particles as the means to identify each member of a particle set to which a specific capture probe has been immobilized. Another exemplary platform uses holographic barcodes to identify cylindrical glass particles. For example, Chandler et al. (U.S. Patent No. 5,981,180) describes a particle-based system in which different particle types are encoded by mixtures of various proportions of two or more fluorescent dyes impregnated into polymer particles. Soini (U.S. Patent No. 5,028,545) describes a particle-based multiplexed assay system that employs time-resolved fluorescence for particle identification. Fulwyler (U.S. Patent No. 4,499,052) describes an exemplary method for using particle distinguished by color and/or size. U.S. Publication Nos. 2004-0179267, 2004-0132205, 2004-0130786, 2004-0130761, 2004-0126875, 2004-0125424, and 2004-0075907 describe exemplary particles encoded by holographic barcodes.

U.S. Patent No. 6,916,661 describes polymeric microparticles that are associated with nanoparticles that have dyes that provide a code for the particles. The polymeric microparticles can have a diameter of less than one millimeter, e.g., a size ranging from about 0.1 to about 1,000 micrometers in diameter, e.g., 3-25 $\mu$m or about 6-12 $\mu$m. The nanoparticles can have, e.g., a diameter from about 1 nanometer (nm) to about 100,000 nm in diameter, e.g., about 10 - 1,000 nm or 200 - 500 nm.

Also described herein are kits containing any of the nucleic acid arrays described herein. The kits can, optionally, contain instructions for detecting and/or measuring the level of one or more genes in a biological sample.

The kits can optionally include, e.g., a control biological sample or control labeled-amplicon set containing known amounts of one or more amplicons recognized by nucleic acid probes of the array. In some instances, the control can be an insert (e.g., a paper insert or electronic medium such as a CD, DVD, or floppy disk) containing expression level ranges of one or more genes predictive of a response to an anti-TNF therapy.

The kits can include one or more reagents for processing a biological sample. For example, a kit can include reagents for isolating mRNA or genomic DNA from a biological sample and/or reagents for amplifying isolated mRNA (e.g., reverse transcriptase, primers for reverse transcription or PCR amplification, or dNTPs) and/or genomic DNA. The kits can also, optionally, contain one or more reagents for detectably-labeling an mRNA, mRNA amplicon, genomic DNA or DNA amplicon, which reagents can include, e.g., an enzyme such as a Klenow fragment of DNA polymerase, T4 polynucleotide kinase, one or more detectably-labeled dNTPs, or detectably-labeled gamma phosphate ATP (e.g., $^{33}$P-ATP).

The kits can include a software package for analyzing the results of, e.g., a microarray analysis or expression profile.

The kits can also include one or more antibodies for detecting the protein expression of any of the genes described herein. For example, a kit can include (or in some cases consist of) a plurality of antibodies capable of specifically binding to one or more proteins encoded by any of the genes depicted in Table 1 and optionally, instructions for detecting the one or more proteins and/or a detection antibody comprising a detectably-labeled antibody that is capable of binding to at least one antibody of the plurality. The kits can include antibodies that recognize at least two (e.g., three, four, five, six, seven, eight, nine, 10, 11, 12, 13, 14, 15, 16, 17, 18, 19, 20, 21, 22, 23, or 24) proteins encoded by genes depicted in Table 1.

The kits described herein can also, optionally, include instructions for administering an anti-TNF therapy where the expression level of one or more genes, detectable by the array predicts that a subject will response to an anti-TNF therapy. The kits can contain instructions for administering a variety of non-anti-TNF therapies where the expression level of one or more genes, detectable by the array predicts that a subject will not respond to an anti-TNF therapy.

The following are examples of the practice of the invention. They are not to be construed as limiting the scope of the invention in any way.

**EXAMPLES**

Example 1. Whole blood gene expression profiles from RA patients predict non-response to anti-TNFs therapy

**Clinical Evaluation of Patients.** Human patients with active disease fulfilling the American College of Rheumatology (ACR) criteria and were previously naive to anti-TNF agents were enrolled in the study. 116 patients beginning anti-TNF therapies (54 etaneracept, 25 adalimumab, 37 infliximab) were consented for this study. Clinical data and baseline gene expression profiles were available for 75 of these patients. The inclusion criteria were: 18 years of age or older, meeting the 1987 ACR criteria, disease activity of at least 6 swollen joints, no previous exposure to anti- TNF medication for a six month duration. The exclusion criteria for our study were those who take 10 mg or more oral steroid therapy per day at the time of enrollment in this study. Peripheral blood samples were collected to isolate RNA using PaxGene tubes. The clinical information required for evaluating response to therapy was also recorded for each patient at baseline and 14 weeks post-treatment. The blood of 42 healthy controls was also collected and profiled using the same procedures. Written informed consent was obtained from all subjects.

**Blood sample acquisition and RNA processing.** Peripheral blood was collected directly into Paxgene tubes according to manufacturer's specifications (Qiagen, Valencia, CA). Blood was collected from RA patients during three visits: pre-treatment, 6 weeks, and 14 weeks post-treatment. Peripheral blood from 89 healthy control patients, for a single time point, was also collected into Paxgene tubes. Total RNA was extracted using the RNeasy kit according to manufacturer's specifications (Qiagen, Valencia, CA). For detailed methods see, e.g., Batliwalla et al, 2005.

**RNA labeling and microarray hybridization.** Sample labeling and hybridization were carried out using the NuGEN Ovation Biotin systems (NuGEN Technologies, Inc, San Carlos, CA) as described in the NuGEN Ovation Biotin system for 96 well plates (version 5). Washing, staining, and scanning of the hybridized arrays was completed as described in the Eukaryotic Target Preparation protocol in the Affymetrix® expression analysis technical manual (702064 rev 2) for Genechip® cartridge arrays using the Genechip® Array Station (Affymetrix, Santa Clara, CA). In summary, 20ng of PaxGene (preAnalytiX GmbH, Hombrechtikon, Germany) purified total RNA in $2\mu$L was annealed with $2\mu$L "first strand primer mix" (A1) at 65°C for 5 minutes and chilled on ice. The annealed template was incubated at 48°C for 60 minutes with $6\mu$L of first strand master mix (A2 and A3) and then chilled to 4°C. To the completed first stand reaction, $10\mu$L of second strand master mix (B1 and B2) was added and the reaction incubated at 37°C for 30 minutes, 75°C for 15 minutes, and then cooled to 4C. To amplify cDNA, $120\mu$L of SPIA™ master mix (Nugen, San Carlos, CA) was added the $20\mu$L completed second strand reaction and the reaction incubated at 48°C for 60 minutes. The amplified cDNA was purified in using the 96 well dye terminator removal kit (AB gene, Surrey, UK-cat# AB-0943/A) according to the manufacturer's recommendations. To fragment the purified and amplified cDNA, $5\mu$L of fragmentation buffer (F1) and enzyme mix (F2) was added to $25\mu$L and the reaction incubated at 50°C for 30 minutes and then chilled to 4C. Biotin labeling of the fragmented cDNA was completed by addition $5\mu$L biotin labeling buffer (F3) and $2.5\mu$L of labeling enzyme mix (F4) with incubation at 50°C for 30 minutes and then cooled to 4C. The fragmented and labeled cDNA was then purified using the 96 well dye terminator removal kit (AB gene, Surrey, UK-cat# AB-0943/A) according to the manufacturer's recommendations. Three $\mu$g of fragmented labeled cDNA was resuspended in 300 $\mu$L 1X hybridization buffer containing 100 mM MES (2-(N-morpholino)ethanesulfonic acid), 1 M [Na+], 20 mM EDTA, 0.01% Tween 20, 0.5 mg/mL Aceylated BSA, 0.1 mg/mL herring sperm DNA, control oligo B2, and control transcripts bioB 1.5 pM, bioC 5 pM, bioD 25 pM, and cre 100 pM, and hybridized to Human Genome U133 plus 2.0 Genechip® probe arrays according to manufacturer's protocol (Affymetrix®, Santa Clara, CA). The hybridized GeneChip® probe arrays were washed and stained using Streptavidin-Phycoerythrinin (Molecular Probes, Eugene, OR) and amplified with biotinylated anti-streptavidin (Vector Laboratories, Burlingame, CA) (Sigma, Saint Louis, MO) GeneChip® Fluidics Station 400 (Affymetrix, Santa Clara, CA) using an antibody amplification protocol. The GeneChip® probe arrays were scanned using GeneArray Scanner 3000 (Hewlett Packard, Corvallis, OR).

**Gene Expression Data Acquisition and analysis.** Following GeneChip hybridization and scanning, signals from the scans were normalized using the CGRMA algorithm (Li et al. (2001) Genome Biol 2, RESEARCH0032; Irizarry et al. (2003) Nucleic Acids Res 31: e15; and Wu et al. (2004) Journal of the American Statistical Association 99: 909-917). All calculations were performed using R and Bioconductor computational tools (Genetelman et al. (2005) Bioinformatics and Computational Biology Solutions using R and Bioconductor (Springer)). Development of predictors for anti-TNF response from gene expression required several data reduction steps. To identify genes correlated with anti-TNF response over time, the general linear modeling tool implemented in R (Genetelman et al. (2005) Bioinformatics and Computational Biology Solutions using R and Bioconductor (Springer)) was used. The significance of the linear model was assessed by t- statistic and for each random sample selection and gene this t-value was recorded. To further characterize differentially expressed genes, genes were identified that have significantly different expression levels between any of the three patient groups: non-responders, medium, responders and the normal controls, plus those genes that are significantly different between non-responders and responders. A linear modeling approach was applied to the patient data grouping the samples into those 4 pre-defined groups. Standard linear Modeling data analysis approach (MANOVA) was applied to identify genes with a significantly different expression. The significance of each difference was evaluated using t-statistics. In order to take into account the multiple hypotheses testing effect, the significance of the fold changes were evaluated using the Bayesian analysis of variations in the samples, groups, and genes (Smyth, G. K. (2004) Stat. Appl. Genet. Mol. Biol. 3, Article3). The genes with a significance p-value< 0.05 of Bayesian posterior probability of being differentially expressed were selected. The selection also required that the genes have fold changes of at least 1.5.

When sets of suitable transcripts were identified from the data reduction methods, they were further selected to include only genes with biological annotation in publicly available genome databases. This gene set was used to develop predictors of anti-TNF response using the Random Forest machine learning method (Breiman, L. (2001) Machine Learning 45: 5-32). For each patient identified as a "RESPONDER" or "NON-RESPONDER," gene expression levels were determined by GeneChip® RMA (GCRMA; Affymetrix®). The Random Forest approach builds a number of decision trees from randomly selected k-genes and about 2/3 of the patient samples. The algorithm repeats this process many times while a priori user can choose any k to select random k variables for each tree. For each variable (gene expression) the Random Forest approach calculates its importance to the predictions. Given the randomness of the forest construction, the importance of a gene is not a deterministic value. That is the importance of a gene can vary depending on the content of the forest.

**Initial data processing and gene filtering.** Gene expression profiles from whole blood collected pre-treatment from 116 RA patients using the Affymetrix HGU133Plus2 chip. In addition to the RA patients we have collected and profiled blood from 42 healthy controls. The patients were treated with anti-TNF drugs (Remicade, Enbrel, Humira). Disease severity was evaluated using the DAS28 score and EULAR classification (Fransen et al. (2005) Clin Exp Rheumatol 23: S93-99). Patients were followed up 8 weeks and 14 weeks after the baseline visit and blood was collected at those 3 visits. Patients' disease score was assessed at those visits. Typically patients with moderate to severe RA are treated with anti-TNF and in consequence in our patient cohort there were no good responders only non-responders and moderate responders. According to EULAR classification good responders are patients with mild disease (DAS28 < 3.2) who improved their DAS28 by at least 40% when compared to the baseline.

The 14 weeks after baseline visit (visit3) was selected to identify in our cohort the good responders in our group as it is a common time-point for making clinical decisions about effectiveness of therapy. The DAS28 score improvement was calculated for each patient using the following formula:

$$\Delta DAS28 = \%(DAS28visit1 - DAS28visit3).$$

Following the EULAR classification (van Gestel et al. (1998), *supra*), responders, intermediate responders, and non-responders were defined. For the purpose of predictor development, the patient cohort can thus consist of three groups: non-responders (22 patients), intermediate responders (29 patients), and responders (24 patients).

Samples from all patients and healthy controls were processed using first GCRMA data normalization method (Li et al. (2001) Genome Biol 2, RESEARCH0032; Irizarry et al. (2003) Nucleic Acids Res 31: e15; and Wu et al. (2004) Journal of the American Statistical Association 99: 909-917). Data was also independently processed using RMA (Li et al. (2001) Genome Biol 2, RESEARCH0032; Irizarry et al. (2003) Nucleic Acids Res 31, e15; and Wu et al. (2004) Journal of the American Statistical Association 99: 909-917). All calculations were done using R and Bioconductor computational tools (see, e.g., Genetelman et al. (2005) Bioinformatics and Computational Biology Solutions using R and Bioconductor (Springer)).

As the preliminary step of data reduction the MAS5 algorithm (as implemented in Bioconductor) was used to identify transcripts present in the samples. It was required that transcripts were called present with p.value < 0.01 in at least 50% of the samples belonging to one of the four groups: controls, responders, intermediate responders and non-responders. Thus the subsequent analysis was applied to 23,686 transcripts considered as present.

The first step of data reduction identified all baseline gene expressions that significantly correlated with relative DAS28 score. The general linear modeling tool implemented in R (Gentelman et al., *supra*) was used to model ΔDAS28 scores by gene expression values using all baseline samples: medium, responders and non-responders. The significance of the linear model fit was assessed by t-statistic. For each gene and a random selection of 90% of samples a t-value was recorded. The random sampling and fitting procedure were repeated 100 times for each gene. From the significance of those 100-modelling steps, the mean significance for each gene was calculated by removing the top and bottom 5% of the highest and lowest t-values. In addition, patient responses were randomly permuted 100 times and fitted the expression values to the permuted responses. The t.values of the permuted fits generated a distribution of expected by chance fit. Using this random distribution, the fit of the true response values was evaluated to determine if it was significantly different form a random fit by more then two standard deviations. 894 gene probes were selected as correlating significantly with relDAS28 score with the p-value < 0.025 for the fit (t.value better then 2), and significant when compared to fits generated with permuted response. Since these calculations are CPU intensive, the data was processed using the 100 nodes (200 CPU) LINUX cluster.

As a second step in our evaluation of the candidate genes we have identified those that have significantly different expression between any of the three patient groups: non-responders, medium, responders and the normal controls, plus those genes that are significantly different between non-responders and responders. We have applied a linear modeling approach to patient data grouping the samples into those 4 pre-defined groups. We have then applied standard linear Modeling data analysis approach (MANOVA) to identify genes with a significantly different expression. The significance of each difference was evaluated using t-statistics. In order to take into account the multiple hypotheses we further evaluated the significance of the fold changes using the Bayesian analysis of variations as implemented by the limma package (Smyth, G. K. (2004) Stat Appl Genet Mol Biol 3, Article3). Genes with a significance p-value < 0.05 of Bayesian posterior probability of being differentially expressed gene and with at least 1.5 fold difference were selected. The total number of gene-probes determined as significantly differentially expressed with above requirements was 7,972.

From the 894 genes that were significantly correlated with relDAS28 score 291 genes were also considered as being differentially expressed by selection described above. We further selected from those 291 gene probes 166 probes that had the log2 expression value as estimated by the GCRMA of at least 6 and only those genes that have been reviewed in the ENTREZ NCBI human genome database. That is we chose only those genes that had a name assigned and excluded probe sets not associated with an annotated gene or an open reading frame pending human review.

**Finding optimum parameters for random forest.**

The 166 gene probes constituted the starting point for construction of the predictor of the anti-TNF response between the responder and non-responder patients. The random forest (Brieman (2001) Machine Learning 45: 5-32) machine learning method was applied to these probes to identify the best predictor or set of predictors.

Two parameters that are important in random forest algorithm are number of trees used in the forest *(ntree)* and number of random variables used in each split *(mtry).* To find the number of trees required for a stable random forest classifier, the random forest test was run with different *ntree* values *i.e. ntree* = {1, 500, 1000...., 120,000}. 10 runs were performed for each *ntree* value, recorded OOB error rate and calculated the median and standard deviation of error rate. The optimum *ntree* was selected for which the standard deviation of error stabilized.

To find the optimum *may,* a similar procedure was applied such that RF was run 10 times for a range of *mtry* values which consist of multiples of the default *mtry* $(\sqrt{\text{number of variables}} = 22)$ starting from 0.05x*mtry* up to *mtry* = number of samples with increments of 0.05. The OOB error rate was recorded was each run. Optimum *mtry* value(s) were selected for which the error rate has stabilized and reached to minimum. In particular, *mtry* values were first ranked according to OOB error rate then ranked based on the following formula:

$$\text{abs}(\text{m.OOB}[mtry_i] - \text{m.OOB}[mtry_{i-1}) + \text{abs}(\text{m.OOB}[mtry_{i+1}] - \text{m.OOB}[mtry_i]) + \text{std.OOB}[mtry_i]$$

$$(i = 2, n),$$

where m.OOB[$mtry_i$] is the median OOB error rate for ith mtry, n is the total number of *mtry* values, and std.OOB is the standard deviation of the error rate. *mtry* values that satisfy both the lowest error rate and the highest stability (lowest value from the equation) criteria were selected as optimum values.

**Selecting genes consistently important in prediction.** A "convergent random forest" algorithm was developed in order to select genes that are consistently important in prediction. The steps of the algorithm are:

1. Run random forest 10 times.
2. Select genes that are considered important in all 10 runs (variable importance > 0)
3. Repeat 1-2 until the number of genes selected does not change.
4. Repeat 1-3 for different *mtry* values selecting a "convergent" gene set for each *mtry* value.
5. Select the common genes between the 'convergent' gene sets.

**Selecting minimum number of genes with maximum prediction accuracy.** A series of steps were carried out to find a non-redundant set with minimum number of genes and maximum accuracy using selection techniques based on importance ranking and clustering. The selection techniques and the whole workflow are outlined below.

### Selection based on importance ranking

1. Rank the genes according to variable importance in decreasing order: $g_1, g_2, .., g_n$
2. Set x = 1.
3. Select x top genes, run RF and record OOB error.
4. Set x = x + 1. Repeat from Step 3 until x = n.
5. Select x which has minimum OOB error rate.

### Selection based on clustering

1. Cluster the genes using the correlation of the gene expression values as the distance matrix.
2. Set k = 2.
3. Cut the tree into k clusters. From each cluster select the gene with the highest variable importance (k genes in total).
4. Set k = k+1. Repeat from Step 3 until k = x (see *Selection based on importance ranking*)
5. Select k-genes which have minimum OOB error rate.

### Complete procedure to select minimum genes:

for *i* = 1:50 {

●  Select genes based on importance ranking: predictive_gene_set$_{i,1}$. Record x as x$_i$.
●  Select genes based on clustering: predictive_gene_set$_{i,2}$. Record k as k$_i$.

}

●  Combine predictive_gene set$_{i,1}$ and predictive_gene_set$_{i,2}$.
●  Calculate Gene Predictive Index (GPI) for each gene g:

$$GPI(g) = \left( \sum_i \frac{1 - OOB[x_{\min}]}{\left| G_i^{x_{\min}} \right|} + \sum_i \frac{1 - OOB[k_{\min}]}{\left| G_i^{k_{\min}} \right|} \right) / \max(i)$$

where NPG is the Number of Predictive Genes in run i where gene g is selected either by importance ranking or by clustering. Where $OOB[x_{\min}]$ is the minimal error rate and $\left| G_i^{x_{\min}} \right|$ the number of best genes selected by importance. Similarly, $OOB[k_{\min}]$ is the minimal error rate and $\left| G_i^{k_{\min}} \right|$ the number of genes selected by cluster cutting. Thus NPG$_i$ = x$_i$ if gene g is selected by importance ranking and NPG$_i$ = k$_i$ if it is selected by clustering. Thus if the number of predictive genes including gene g identified in that run is small, that gene is given a higher index. Moreover, as the frequency of appearance of the gene in the runs increases, GPI of the gene also increases (through summation).
●  Rank the genes according to GPI in decreasing order.
●  Perform steps 2-5 outlined in *Selection based on importance ranking.*

**Different classification algorithms.** A final set of genes were run with different classification algorithms to compare the performances with respect to random forest. k nearest neighbor classifier (kNN) is run with k ∈ {1, 3, 5, ...,

21} and k with the smallest error was chosen. SVM with radial kernel was run with combinations of cost parameter c ∈ {1, 2, 4} and the parameter y ∈ {$2^{-1}$/18, 1/18, 2/18} as suggested by Gentleman et al. (Bioinformatics and Computational Biology Solutions using R and Bioconductor. New York, Springer). A random forest test was run with 100,000 trees and default *mtry.* The analysis was performed using MLInterfaces package in R (Bioconductor).

Example 2. Construction of a Stable Predictor with Minimum Number of Genes and Maximum Performance

Initial data processing and gene filtering resulted in 166 gene probes which are significantly correlated with the anti-TNF response and differentially expressed between responders and non-responders as well as between patients and normal controls. Starting from 166 gene probes, the goal was to find a stable predictor with minimum number of genes and maximum performance.

This goal was achieved by completing two tasks: (1) To construct a stable random forest classifier and (2) to select 'important' yet minimum number of genes that should be used in the final predictor. Each of these tasks is elaborated on below in detail.

**1. Constructing a stable random forest classifier**

The following two questions were addressed to construct a stable RF classifier: How many trees are needed in the forest to have a stable classifier? How many random variables should be selected randomly per split?

First, the optimum number of trees for which a stable classifier exists was determined i.e., where the performance does not change as more trees are added to the forest. For this a random forest was run 10 times for different *ntree* values, each time recording OOB error rate. Then, the median and the standard deviation (std) across these 10 runs was calculated. The Error rate quickly stabilized while the standard deviation continued to decline as more trees were added to the forest. Therefore, an *ntree* after which the standard deviation stabilized was determined. This procedure gives an initial estimate of the optimum *ntree* for a dataset however it should not be taken as a static value. The researcher should continually explore the robustness of the results as the dataset (number of genes and samples) changes and add more trees when necessary.

The next question was how the performance changes with *mtry.* For this, RF was run 10 times for a range of *mtry* values recording OOB error rate at each run. The change of median and standard deviation of error rate with *mtry* is shown for *ntree* = 40,000. Since an *ntree* for which the standard deviation stabilized was selected at the previous step, the standard deviation does not change at all with *mtry.* After ranking *mtry* values based on error rate and stability, one can select an optimum *mtry* or a range of *mtry* values that are reasonably different from each other *(e.g.* ≥ 1.5 x previous *mtry*). The results proved that *mtry* effects the performance and it is therefore important to experiment different values and evaluate the consistency of the results.

The procedure was repeated with different *ntree* values. The change of OOB error rate with *mtry* is independent of *ntree* (see, e.g., Diaz-Uriarte et al. (2006) BMC Bioinformatics 7 (3)). Alternatively, the results can imply that the *ntree* range determined in the previous step is really optimum such that adding more trees has no effect on the error rate for the same *mtry* values.

**Gene selection.** After the optimum parameters have been determined for the random forest classifier, the next goal was to select genes that will be used in the final predictor. The final goal was to have a predictor with minimum number of genes and maximum prediction accuracy. Therefore, a set of genes that were informative, non-redundant, and consistently important in prediction were selected as described below.

*Selecting genes consistently important in prediction*

A frequency-based gene selection algorithm that takes into account both the variable importance and consistency was selected. The 'convergent random forest' algorithm (see Materials and Methods II) was run using an *mtry* value of *mtry* = {45}. The algorithm resulted in a "convergent" set of 40 genes. The performance of this gene set was calculated with 10 independent RF runs. Overall, the classifier with 40 genes selected by convergence resulted in an improved accuracy over the initial 166 gene set. The 40 genes are shown in Table 2.

Table 2.

| Gene Symbol | Log2 Fold difference Between Non-responders and responders | p-value | Log2 expression in healthy controls | Log2 expression in RA Moderate patients visit 1 | Log2 expression in RA non-responder patients visit 1 | Log2 expression in RA responder patients visit 1 |
|---|---|---|---|---|---|---|
| CLTB | -0.67 | 2.12E-04 | 5.68 | 5.96 | 5.85 | 6.52 |
| HDAC4 | 0.38 | 4.74E-04 | 8.65 | 9.13 | 9.34 | 8.96 |
| CXorf52 | 0.51 | 5.05E-04 | 8.32 | 7.89 | 8.13 | 7.62 |
| RBBP4 | -0.5 | 8.30E-04 | 6.49 | 5.99 | 5.80 | 6.30 |
| COL4A3BP | -0.45 | 9.20E-04 | 6.53 | 5.84 | 5.64 | 6.09 |
| C9orf80 | -0.46 | 9.79E-04 | 7.03 | 6.05 | 5.73 | 6.19 |
| ANKRD12 | 0.69 | 1.29E-03 | 7.24 | 7.86 | 8.08 | 7.39 |
| CAMK2G- | 0.58 | 1.45E-03 | 6.92 | 5.98 | 5.75 | 6.33 |
| PCBP2 | -0.37 | 1.55E-03 | 9.59 | 8.09 | 7.97 | 8.34 |
| COL4A3BP | -0.48 | 1.57E-03 | 6.54 | 5.87 | 5.60 | 6.08 |
| YIPF6 | -0.41 | 1.85E-03 | 8.29 | 7.21 | 6.88 | 7.28 |
| MYLIP | -0.52 | 2.58E-03 | 6.87 | 5.87 | 5.98 | 6.50 |
| ZNF294 | -0.27 | 4.78E-03 | 9.26 | 8.46 | 8.35 | 8.62 |
| RER1 | -0.26 | 4.79E-03 | 8.23 | 8.75 | 8.65 | 8.92 |
| CALM2 | -0.47 | 5.18E-03 | 5.51 | 6.75 | 6.58 | 7.05 |
| ARF5 | -0.42 | 5.96E-03 | 6.77 | 7.31 | 7.21 | 7.63 |
| ARF1 | -0.45 | 6.01E-03 | 6.05 | 5.45 | 5.21 | 5.66 |
| HDAC5 | -0.38 | 6.06E-03 | 5.38 | 5.87 | 5.74 | 6.11 |
| CASP5 | 0.9 | 6.34E-03 | 4.78 | 5.70 | 6.35 | 5.45 |
| MXRA7 | 0.87 | 6.39E-03 | 5.45 | 6.26 | 6.55 | 5.68 |
| ANKIB1 | -0.38 | 6.89E-03 | 6.37 | 4.73 | 4.53 | 4.91 |
| BRWD2 | -0.61 | 7.84E-03 | 6.16 | 4.85 | 4.59 | 5.20 |
| FAM44A | 0.54 | 8.04E-03 | 9.22 | 7.69 | 8.00 | 7.45 |
| PGK1 | -0.31 | 9.75E-03 | 8.11 | 8.91 | 8.79 | 9.10 |
| ZFP36LI | -0.88 | 9.95E-03 | 9.75 | 7.71 | 7.42 | 8.30 |
| SERF2 | -0.39 | 1.11E-02 | 9.14 | 9.89 | 9.72 | 0.11 |
| SERF2 | -0.39 | 1.18E-02 | 8.22 | 8.71 | 8.63 | 9.02 |
| SRGAP2 | -0.52 | 1.42E-02 | 6.37 | 4.18 | 4.03 | 4.55 |
| MNT | 0.26 | 1.79E-02 | 7.95 | 8.46 | 8.61 | 8.34 |
| SEL1L | 0.38 | 2.55E-02 | 10.00 | 19.50 | 19.68 | 9.30 |
| TUG1 | -0.27 | 2.72E-02 | 9.35 | 7.95 | 7.88 | 8.15 |
| LGALS9 | -0.44 | 2.81E-02 | 6.20 | 6.58 | 6.63 | 7.07 |
| SLC25A39 | -0.68 | 3.46E-02 | 9.06 | 9.02 | 8.25 | 8.93 |
| EEA1 | 0.32 | 3.70E-02 | 6.63 | 5.90 | 6.01 | 5.68 |

(continued)

| Gene Symbol | Log2 Fold difference Between Non-responders and responders | p-value | Log2 expression in healthy controls | Log2 expression in RA Moderate patients visit 1 | Log2 expression in RA non-responder patients visit 1 | Log2 expression in RA responder patients visit 1 |
|---|---|---|---|---|---|---|
| EGLN2 | -0.26 | 5.97E-02 | 7.27 | 7.92 | 7.91 | 8.17 |
| RPA3 | -0.34 | 6.47E-02 | 7.56 | 6.63 | 6.39 | 6.73 |
| FOXJ3 | 0.24 | 6.76E-02 | 8.75 | 8.00 | 8.23 | 7.99 |
| DNAH1 | 0.24 | 8.55E-02 | 5.03 | 6.00 | 6.28 | 6.04 |
| PTCH1 | 0.48 | 1.55E-01 | 7.74 | 6.76 | 6.70 | 6.22 |
| SFRS2 | 0.15 | 1.74E-01 | 12.41 | 11.86 | 11.97 | 11.82 |

For the selection of the most accurate and non-redundant set of genes that differentiate between responders and non-responders. First, how the error rate changes with the number of genes selected by the importance measure was explored. For this, RF was run with the first ranked gene, then the second gene was added, and so on until all 166 genes were added sequentially. The step-wise increase of the error rate was most probably due to the correlations between the genes. As the aim was to select a minimum number of non-redundant genes, a clustering procedure was developed to select uncorrelated, more informative, and less noisy gene set.

Starting with the convergent gene set (40 genes), first the procedure described above was repeated i.e., the change of error rate was calculated with the number of genes selected by the importance measure. A minimum error rate (11%) was obtained for the top 24 genes. The top 24 genes were as follows: ANKIB1, ARF1, ARF5, C9orf80, CALM2, CASP5, CLTB, COL4A3BP, CXorf52, DNAH1, EEA1, EGLN2, FAM44A, HDAC4, HDAC5, LGALS9, MXRA7, PGK1, RBBP4, RER1, SEL1L, SERF2, SFRS2, and YIPF6. Then, 40 genes were clustered using the hierarchical clustering with the correlation between gene expressions as a measure of distance. Starting from the clustering tree, the tree was recursively cut into k = 2, 3, 4, 5.... up to the number of genes for which minimum error is obtained by importance ranking. Once the k-subclusters of genes were identified, genes were selected from those subclusters that have been judged as most important for classification. For k-subclusters, k-best genes for each k selection were selected. The performance of the RF predictors constructed from k-best genes is shown in Table 3. The selection of k = 8 resulted in the best performance of the RF predictor with the accuracy of 89%. The best 8 genes were: CLTB, COL4A3BP, CXorf52, FAM44A, MXRA7, PGK1, SFRS2, and YIPF6. Different sets of k-best genes are selected in each run and the union of all those is a set of 24 best genes. The RF predictor build from all best genes has the accuracy of 87%.

Table 3. Performance of the two best gene predictor sets. The prediction was done with the RF predictor and the accuracies were calculated with the OOB cross-validation.

| Gene set | Predicted | Non-responder | Responder | |
|---|---|---|---|---|
| 8-genes | NON-RESPONDER | 20 | 2 | Specificity =91% |
| | RESPONDER | 3 | 21 | Sensitivity =88% |
| | Overall accuracy is 89% | NPV = 91% | PPV = 88% | |
| 24-genes | NON-RESPONDER | 19 | 3 | Specificity =86% |
| | RESPONDER | 3 | 21 | Sensitivity =88% |
| | Overall accuracy is 83% | NPV = 86% | PPV = 88% | |

**Claims**

1. A method of predicting the response of a subject to a therapy comprising an anti-TNF agent, the method comprising:

    providing a biological sample obtained from a subject that has an immune disorder; and

measuring the expression level of the gene ANKIB1 (SEQ ID NO: 1) in the biological sample,
wherein an elevated expression level, as compared to a healthy individual, of ANKIB1 (SEQ ID NO: 1) predicts that the subject will respond to a therapy comprising an anti-TNF agent.

2. The method of claim 1, comprising:

determining that the expression level ANKIB1 (SEQ ID NO: 1) is elevated, as compared to a healthy individual; and
selecting a therapy comprising an anti-TNF agent for the subject.

3. The method of claims 1 or 2, wherein in addition to an elevated expression level of ANKIB1 (SEQ ID NO:1), (i) an elevated expression level, as compared to a healthy individual, of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), or ZFP36L1 (SEQ ID NO: 37), or (ii) a reduced expression level, as compared to a healthy individual, of ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), or SFRS2 (SEQ ID NO: 32) predicts that the subject will respond to a therapy comprising an anti-TNF agent.

4. The method of claims 1 or 2, wherein the method comprises measuring the expression level of at least eight genes selected from ANKIB1 (SEQ ID NO: 1) and one or more further genes selected from the group consisting of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), and SFRS2 (SEQ ID NO: 32).

5. The method of claim 4, wherein the at least eight genes comprise ANKIB1 (SEQ ID NO: 1) and seven or more of ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), and YIPF6 (SEQ ID NO: 36).

6. The method of claims 1 or 2, wherein the method comprises measuring the expression level of at least 24 genes selected from ANKIB1 (SEQ ID NO: 1) and one or more further genes selected from the group consisting of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEO ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), and SFRS2 (SEQ ID NO: 32).

7. The method of claim 6, wherein the at least 24 genes comprise ANKIB1 (SEQ ID NO: 1) and additionally ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32) and YIPF6 (SEQ ID NO: 36).

8. A method of predicting the response of a subject to a therapy comprising an anti-TNF agent, the method comprising:

providing a biological sample obtained from a subject that has an immune disorder; and
measuring the expression level of the gene ANKIB1 (SEQ ID NO: 1) in the biological sample,
wherein a reduced expression level, as compared to a healthy individual, of ANKIB1 (SEQ ID NO: 1) predicts that the subject will not respond to a therapy comprising an anti-TNF agent.

9. The method of claim 8, comprising:

determining that the expression level of ANKIB1 (SEQ ID NO: 1) is reduced, as compared to a healthy individual; and
selecting a therapy comprising a non-anti-TNF agent for the subject.

10. The method of claims 8 or 9 wherein in addition to an reduced expression level of ANKIB1 (SEQ ID NO:1), (i) an elevated expression level, as compared to a healthy individual, of ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), or SFRS2 (SEQ ID NO: 32), or (ii) a reduced expression level, as compared to a healthy individual, of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), or ZFP36L1 (SEQ ID NO: 37) predicts that the subject will not respond to a therapy comprising an anti-TNF agent.

11. The method of claims 8 or 9, wherein the method comprises measuring the expression level of at least eight genes selected from ANKIB1 (SEQ ID NO: 1) and one or more further genes selected from the group consisting of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), and SFRS2 (SEQ ID NO: 32).

12. The method of claim 11, wherein the at least eight genes comprise ANKIB1 (SEQ ID NO: 1) and seven or more of ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), and YIPF6 (SEQ ID NO: 36).

13. The method of claims 8 or 9, wherein the method comprises measuring the expression level of at least 24 genes selected from ANKIB1 (SEQ ID NO: 1) and one or more further genes selected from the group consisting of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), and SFRS2 (SEQ ID NO: 32).

14. The method of claim 13, wherein the at least 24 genes comprise ANKIB1 (SEQ ID NO: 1) and additionally ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ 10 NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32) and YIPF6 (SEQ ID NO: 36).

15. The method of any of the preceding claims, wherein (i) the RNA level of the gene or genes is measured, or (ii) the protein level of the gene or genes is measured.

16. An anti-TNF agent that inhibits the activity of TNF for use in treating an immune disorder in a subject, wherein the subject is identified by the method of claim 1 as a subject who will respond to a therapy comprising an anti-TNF agent.

17. The anti-TNF agent for use as in claim 16, wherein the subject has been identified as having elevated or reduced expression levels, as compared to a healthy individual, of at least eight genes selected from ANKIB1 (SEQ ID NO: 1) and one or more further genes selected from the group consisting of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), and SFRS2 (SEQ ID NO: 32).

18. The anti-TNF agent for use as in claim 17, wherein the at least eight genes comprise ANKIB1 (SEQ ID NO: 1) and seven or more of ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), and YIPF6 (SEQ ID NO: 36).

19. The anti-TNF agent for use as in claim 17, wherein the at least eight genes comprise CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32), and YIPF6 (SEQ ID NO: 36).

20. The anti-TNF agent for use as in claim 16, wherein the subject has been identified as having elevated expression levels, as compared to a healthy individual, of at least 24 genes selected from ANKIB1 (SEQ ID NO: 1) and one or more further genes selected from the group consisting of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), and SFRS2 (SEQ ID NO: 32).

21. The anti-TNF agent for use as in claim 20, wherein the at least 24 genes comprise ANKIB1 (SEQ ID NO: 1) and additionally ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32) and YIPF6 (SEQ ID NO: 36).

22. The method of any of claims 1-15, wherein the expression level of ANKIB1 (SEQ ID NO: 1) is elevated or reduced, as compared to a healthy individual, by at least about 1.5 fold.

23. The anti-TNF agent for use as in any of claims 16-21, wherein the expression level of ANKIB1 (SEQ ID NO: 1) is elevated, as compared to a healthy individual, by at least about 1.5 fold.

24. The method of any of claims 1-15, wherein the method comprises measuring the expression level of at least five genes selected from ANKIB1 (SEQ ID NO: 1) and one or more further genes (i) selected from the group consisting of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), and SFRS2 (SEQ ID NO: 32) or (ii) selected from the group consisting of ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32) and YIPF6 (SEQ ID NO: 36), or (iii) comprising five or more of CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32), or YIPF6 (SEQ ID NO: 36).

25. The anti-TNF agent for use as in any of claims 16-21, wherein the subject has been identified as having elevated expression levels, as compared to a healthy individual, of at least five genes selected from ANKIB1 (SEQ ID NO: 1) and one or more further genes (i) selected from the group consisting of ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), and SFRS2 (SEQ ID NO: 32), (ii) selected from the group consisting of ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), and YIPF6 (SEQ ID NO: 36), or (iii) comprising five or more of CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32), or YIPF6 (SEQ ID NO: 36).

26. The method of any of claims 1-15, 22 or 24, or the anti-TNF agent for use as in any of claims 16-21, 23 or 25, wherein the subject has an inflammatory disorder.

27. The method of any of claims 1-15, 22 or 24, or the anti-TNF agent for use as in any of claims 16-21, 23 or 25, wherein the subject has rheumatoid arthritis or Crohn's disease.

**28.** The method of any of claims 1-15, 22, 24, 26 or 27, or the anti-TNF agent for use as in any of claims 16-21, 23 or 25-27, wherein the subject is a human.

**Patentansprüche**

**1.** Verfahren zur Vorhersage des Ansprechens eines Individuums auf eine Therapie umfassend ein Anti-TNF-Mittel, wobei das Verfahren folgendes umfasst:

Bereitstellen einer biologischen Probe, die aus einem Individuum erhalten wurde, das an einer Immunkrankheit leidet; und
Messen des Expressionslevels des Gens ANKIB1 (SEQ ID NO: 1) in der biologischen Probe;
wobei ein, im Vergleich zu einem gesunden Individuum, erhöhtes Expressionslevel von ANKIB1 (SEQ ID NO: 1) vorhersagt, dass das Individuum auf eine Therapie anspricht, die ein Anti-TNF-Mittel umfasst.

**2.** Das Verfahren nach Anspruch 1, umfassend:

Bestimmen, dass das Expressionslevel von ANKIB1 (SEQ ID NO: 1), im Vergleich zu einem gesunden Individuum, erhöht ist; und
Auswählen einer Therapie umfassend ein Anti-TNF-Mittel für das Individuum.

**3.** Das Verfahren nach Anspruch 1 oder Anspruch 2, wobei zusätzlich zu einem erhöhten Expressionslevel von ANKIB1 (SEQ ID NO: 1), (i) ein im Vergleich zu einem gesunden Individuum erhöhtes Expressionslevel von
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), oderZFP36L1 (SEQ ID NO: 37),
oder (ii) ein im Vergleich zu einem gesunden Individuum reduziertes Expressionslevel von
ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), oderSFRS2 (SEQ ID NO: 32)
vorhersagt, dass das Individuum auf eine Therapie anspricht, die ein Anti-TNF-Mittel umfasst.

**4.** Das Verfahren nach Anspruch 1 oder Anspruch 2, wobei das Verfahren das Messen des Expressionslevels von mindestens acht Genen umfasst, ausgewählt aus ANK1B1 (SEQ ID NO: 1) und einem oder mehreren zusätzlichen Genen, ausgewählt aus der Gruppe bestehend aus
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), und SFRS2 (SEQ ID NO: 32).

**5.** Das Verfahren nach Anspruch 4, wobei die mindestens acht Gene ANKIB1 (SEQ ID NO: 1)
und sieben oder mehr von
ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEC ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), und YIPF6 (SEQ ID NO: 36)
umfassen.

**6.** Das Verfahren nach Anspruch 1 oder Anspruch 2, wobei das Verfahren das Messen des Expressionslevels von mindestens 24 Genen umfasst, die aus ANKIB1 (SEQ ID NO: 1) und einem oder mehreren zusätzlichen Genen ausgewählt sind, ausgewählt aus der Gruppe bestehend aus

ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), und SFRS2 (SEQ ID NO: 32).

**7.** Das Verfahren nach Anspruch 6, wobei die mindestens 24 Gene ANKIB1 (SEQ ID NO: 1) umfassen und weiterhin folgendes umfassen:

ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32) und YIPF6 (SEQ ID NO: 36).

**8.** Verfahren zur Vorhersage des Ansprechens eines Individuums auf eine Therapie umfassend ein Anti-TNF-Mittel, wobei das Verfahren folgendes umfasst:

Bereitstellen einer biologischen Probe, die aus einem Individuum erhalten wurde, das an einer Immunkrankheit leidet; und
Messen des Expressionslevels des Gens ANKIB1 (SEQ ID NO: 1) in der biologischen Probe;
wobei ein, im Vergleich zu einem gesunden Individuum, reduziertes Expressionslevel von ANKIB1 (SEQ ID NO: 1) vorhersagt, dass das Individuum nicht auf eine Therapie anspricht, die ein Anti-TNF-Mittel umfasst.

**9.** Das Verfahren nach Anspruch 8, umfassend:

Bestimmen, dass das Expressionslevel von ANKIB1 (SEQ ID NO: 1) im Vergleich zu einem gesunden Individuum reduziert ist; und
Auswählen einer Therapie, umfassend ein Nicht-anti-TNF-Mittel für das Individuum.

**10.** Das Verfahren nach Anspruch 8 oder Anspruch 9, wobei zusätzlich zu einem reduzierten Expressionslevel von ANKIB1 (SEQ ID NO: 1), (i) ein erhöhtes Expressionslevel, im Vergleich zu einem gesunden Individuum, von ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), oderSFRS2 (SEQ ID NO: 32),
oder (ii) ein reduziertes Expressionslevel, im Vergleich zu einem gesunden Individuum, von
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), oderZFP36L1 (SEQ ID NO: 37)
vorhersagt, dass das Individuum nicht auf eine Therapie anspricht, die ein Anti-TNF-Mittel umfasst.

**11.** Das Verfahren nach Anspruch 8 oder Anspruch 9, wobei das Verfahren das Messen des Expressionslevels von mindestens acht Genen umfasst, die aus ANKIB1 (SEQ ID NO: 1) und einem oder mehreren zusätzlichen Genen ausgewählt sind, ausgewählt aus der Gruppe bestehend aus
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO:

19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), und SFRS2 (SEQ ID NO: 32).

12. Das Verfahren nach Anspruch 11, wobei die mindestens acht Gene ANKIB1 (SEQ ID NO: 1) umfassen und sieben oder mehr von
ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18). HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), und YIPF6 (SEQ ID NO: 36).
umfassen.

13. Das Verfahren nach Anspruch 8 oder Anspruch 9, wobei das Verfahren das Messen des Expressionslevels von mindestens 24 Genen umfasst, ausgewählt aus ANKIB1 (SEQ ID NO: 1) und einem oder mehreren zusätzlichen Genen, ausgewählt aus der Gruppe bestehend aus
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), und SFRS2 (SEQ ID NO: 32).

14. Das Verfahren nach Anspruch 13, wobei die mindestens 24 Gene ANKIB1 (SEQ ID NO: 1) umfassen und zusätzlich folgendes umfassen:

ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32) und YIPF6 (SEQ ID NO: 36).

15. Das Verfahren nach einem der vorhergehenden Ansprüche, wobei (i) das RNA-Level des Gens oder der Gene gemessen wird, oder (ii) das Proteinlevel des Gens oder der Gene gemessen wird.

16. Anti-TNF-Mittel, das die Aktivität von TNF hemmt, zur Verwendung bei der Behandlung einer Immunkrankheit in einem Individuum, wobei das Individuum durch das Verfahren nach Anspruch 1 als ein Individuum identifiziert wird, das auf eine Therapie anspricht, die ein Anti-TNF-Mittel umfasst.

17. Das Anti-TNF-Mittel zur Verwendung nach Anspruch 16, wobei bei dem Individuum festgestellt wurde, dass es, im Vergleich zu einem gesunden Individuum, erhöhte oder reduzierte Expressionslevels von mindestens acht Genen aufweist, ausgewählt aus ANKIB1 (SEQ ID NO: 1), und einem oder mehreren zusätzlichen Genen, ausgewählt aus der Gruppe bestehend aus
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEC ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), und SFRS2 (SEQ ID NO: 32).

18. Das Anti-TNF-Mittel zur Verwendung nach Anspruch 17, wobei die mindestens acht Gene ANKIB1 (SEQ ID NO: 1) und sieben oder mehrere von folgendem umfassen
ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ 10 NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), und YIPF6 (SEQ ID NO: 36).

19. Das Anti-TNF-Mittel zur Verwendung nach Anspruch 17, wobei die mindestens acht Gene folgendes umfassen
CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32), und YIPF6 (SEQ ID NO: 36).

20. Das Anti-TNF-Mittel zur Verwendung nach Anspruch 16, wobei bei einem Individuum festgestellt wurde, dass es, im Vergleich zu einem gesunden Individuum, erhöhte Expressionslevels von mindestens 24 Genen aufweist, ausgewählt aus ANKIB1 (SEQ ID NO: 1), und einem oder mehreren zusätzlichen Genen, ausgewählt aus der Gruppe bestehend aus
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), und SFRS2 (SEQ ID NO: 32).

21. Das Anti-TNF-Mittel zur Verwendung nach Anspruch 20, wobei die mindestens 24 Gene ANKIB1 (SEQ ID NO: 1) umfassen und zusätzlich folgendes umfassen:

ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32) und YIPF6 (SEQ ID NO: 36).

22. Das Verfahren nach einem der Ansprüche 1-15, wobei das Expressionslevel von ANKIB1 (SEQ ID NO: 1), im Vergleich zu einem gesunden Individuum, mindestens etwa 1,5-fach erhöht oder reduziert ist.

23. Das Anti-TNF-Mittel zur Verwendung nach einem der Ansprüche 16-21, wobei das Expressionslevel von ANKIB1 (SEQ ID NO: 1), im Vergleich zu einem gesunden Individuum, mindestens etwa 1,5-fach erhöht ist.

24. Das Verfahren nach einem der Ansprüche 1-15, wobei das Verfahren das Messen des Expressionslevels von mindestens fünf Genen umfasst, ausgewählt aus ANKIB1 (SEQ ID NO: 1) und einem oder mehreren zusätzlichen Genen (i) ausgewählt aus der Gruppe bestehend aus
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), und SFRS2 (SEQ ID NO: 32) oder (ii) ausgewählt aus der Gruppe bestehend aus ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1 L (SEQ ID NO: 30), SERF2 (SEQ ID NO: 31), SFRS2 (SEQ ID NO: 32) and YIPF6 (SEQ ID NO: 36),
oder (iii) umfassend fünf oder mehrere von
CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32),oder YIPF6 (SEQ ID NO: 36).

25. Das Anti-TNF-Mittel zur Verwendung nach einem der Ansprüche 16-21, wobei bei dem Individuum festgestellt wurde, dass es, im Vergleich zu einem gesunden Individuum, erhöhte Expressionslevels von mindestens fünf Genen aufweist, ausgewählt aus ANKIB1 (SEQ ID NO: 1), und einem oder mehreren Genen (i) ausgewählt aus der Gruppe bestehend aus
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 NO: 32), (ii) ausgewählt aus der Gruppe bestehend aus ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), und YIPF6 (SEQ ID NO: 36),oder(iii) umfassend fünf oder mehrere von CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32),oderYIPF6 (SEQ ID NO: 36).

26. Das Verfahren nach einem der Ansprüche 1-15, 22 oder 24, oder das Anti-TNF-Mittel zur Verwendung nach einem der Ansprüche 16-21, 23 oder 25, wobei das Individuum eine Entzündung aufweist.

27. Das Verfahren nach einem der Ansprüche 1-15, 22 oder 24, oder das Anti-TNF-Mittel zur Verwendung nach einem der Ansprüche 16-21, 23 oder 25, wobei das Individuum an rheumatoider Arthritis oder Morbus Crohn leidet.

28. Das Verfahren nach einem der Ansprüche 1-15, 22, 24, 26 oder 27 oder das Anti-TNF-Mittel zur Verwendung nach einem der Ansprüche 16-21, 23 oder 25-27, wobei das Individuum ein Mensch ist.

## Revendications

1. Procédé pour prédire la réactivité d'un sujet à une thérapie comprenant un agent anti-TNF le procédé comprenant les étapes consistant à :

fournir un échantillon biologique obtenu d'un sujet qui a un désordre immunitaire; et
mesurer le niveau d'expression du gène ANKIB1 (SEQ ID NO: 1) dans l'échantillon biologique,
dans lequel un niveau d'expression élevé, en comparaison d'un individu sain, d'ANKIB1 (SEQ ID NO: 1) prédit que le sujet va répondre à une thérapie comprenant un agent anti-TNF.

2. Le procédé selon la revendication 1, comprenant :

la détermination du fait que le niveau d'expression d'ANKIB1 (SEQ ID NO: 1) est élevé, en comparaison d'un individu sain ; et
la sélection d'une thérapie comprenant un agent anti-TNF pour le sujet.

**3.** Le procédé selon la revendication 1 ou 2, dans lequel en plus d'un niveau d'expression élevé d'ANKIB1 (SEQ ID NO: 1), (i) un niveau d'expression élevé, en comparaison d'un individu sain, de ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C90rf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEO ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ou ZFP36L1 (SEQ ID NO: 37),
ou (ii) un niveau d'expression réduit, en comparaison d'un individu sain, de ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), ou SFRS2 (SEQ ID NO: 32)
prédit que le sujet va répondre à une thérapie comprenant un agent anti-TNF.

**4.** Le procédé selon la revendication 1 ou 2, dans lequel le procédé comprend l'étape consistant à mesurer le niveau d'expression d'au moins huit gènes sélectionnés parmi l'ANKIB1 (SEQ ID NO: 1) et un ou plusieurs gènes supplémentaires sélectionnés parmi le groupe consistant en ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ 10 ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SECS ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), et SFRS2 (SEQ ID NO: 32).

**5.** Le procédé selon la revendication 4, dans lequel les au moins huit gènes comprennent ANKB1 (SEQ ID NO : 1) et sept ou plusieurs de ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), et YIPF6 (SEQ ID NO: 36).

**6.** Le procédé selon la revendication 1 ou 2, dans lequel le procédé comprend l'étape consistant à mesurer le niveau d'expression d'au moins 24 gènes sélectionnés parmi l'ANKIB1 (SEQ ID NO: 1) et un ou plusieurs gènes supplémentaires sélectionnés parmi le groupe consistant en ARF1 (SEQ ID NO: 3), ARF5 (SEQ ID NO: 4), BRWD2 (SEQ ID NO: 5), CALM2 (SEQ ID NO: 7), CLTB (SET ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), et SFRS2 (SEQ ID NO: 32).

**7.** Le procédé selon la revendication 6, dans lequel les au moins 24 gènes comprennent l'ANKIB1 (SEQ ID NO: 1) et de plus ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32) et YIPF6 (SEG7 ID NO: 36).

**8.** Procédé pour prédire la réactivité d'un sujet à une thérapie comprenant un agent anti-TNF, le procédé comprenant les étapes consistant à :

fournir un échantillon biologique obtenu d'un sujet qui a un désordre immunitaire; et
mesurer le niveau d'expression du gène ANKIB1 (SEQ ID NO: 1) dans l'échantillon biologique,
dans lequel un niveau d'expression réduit, en comparaison d'un individu sain, d'ANKIB1 (SEQ ID NO: 1) prédit que le sujet ne va pas répondre à une thérapie comprenant un agent anti-TNF.

**9.** Le procédé selon la revendication 8, comprenant :

la détermination du fait que le niveau d'expression d'ANKIB1 (SEQ ID NO: 1) est réduit, en comparaison d'un individu sain ; et
la sélection d'une thérapie comprenant un agent non-anti-TNF pour le sujet.

**10.** Le procédé selon la revendication 8 ou 9, dans lequel en plus d'un niveau d'expression réduit d'ANKIB1 (SEQ ID NO: 1), (i) un niveau d'expression élevé, en comparaison d'un individu sain, de
ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), ou SFRS2 (SEQ ID NO: 32),
ou (ii) un niveau d'expression réduit, en comparaison d'un individu sain, de
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ou ZFP36L1 (SEQ ID NO: 37)
prédit que le sujet ne va pas répondre à un thérapie comprenant un agent anti-TNF.

**11.** Le procédé selon la revendication 8 ou 9, dans lequel le procédé comprend l'étape consistant à mesurer le niveau d'expression d'au moins huit gènes sélectionnés parmi l'ANKIB1 (SEQ ID NO: 1) et un ou plusieurs gènes supplémentaires sélectionnés parmi le groupe consistant en
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), et SFRS2 (SEQ ID NO: 32).

**12.** Le procédé selon la revendication 11, dans lequel les au moins huit gènes comprennent l'ANKIB1 (SEQ ID NO: 1) et sept ou plusieurs de
ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), et YIPF6 (SEQ ID NO: 36).

**13.** Le procédé selon la revendication 8 ou 9, dans lequel le procédé comprend l'étape consistant à mesurer le niveau d'expression d'au moins 24 gènes sélectionnés parmi l'ANKIB1 (SEQ ID NO: 1) et un ou plusieurs gènes supplémentaires sélectionnés parmi le groupe consistant en
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), et SFRS2 (SEQ ID NO: 32).

14. Le procédé selon la revendication 13, dans lequel les au moins 24 gènes comprennent l'ANKIB 1 (SEQ ID NO: 1) et en outre
ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1 (SEQ ID NO: 30), SERF2 (SEQ ID NO: 31), SFRS2 (SEQ ID NO: 32) et YIPF6 (SEQ ID NO: 36).

15. Le procédé selon l'une des revendications précédentes, dans lequel (i) le niveau d'ARN du gène ou des gènes est mesuré, ou (ii) le niveau de protéine du gène ou des gènes est mesuré.

16. Agent anti-TNF qui inhibe l'activité de TNF pour l'utilisation dans le traitement d'un désordre immunitaire dans un sujet, dans lequel le sujet est identifié par le procédé selon la revendication 1 en tant qu'un sujet qui va répondre à une thérapie comprenant un agent anti-TNF.

17. L'agent anti-TNF pour l'utilisation selon la revendication 16, dans lequel le sujet a été identifié par le fait d'avoir des niveaux d'expression élevés ou réduits, en comparaison d'un individu sain, d'au moins huit gènes, sélectionnés parmi l'ANKIB 1 (SEQ ID NO: 1), et un ou plusieurs gènes supplémentaires sélectionnés parmi le groupe consistant en ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SET ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), et SFRS2 (SEQ ID NO: 32).

18. L'agent anti-TNF pour l'utilisation selon la revendication 17, dans lequel les au moins huit gènes comprennent l'ANKIB 1 (SEQ ID NO: 1) et sept ou plusieurs de
ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1L (SEQ ID NO: 30), SERF2 (SEQ ID NO: 31), SFRS2 (SEQ ID NO: 32), et YIPF6 (SEQ ID NO: 36).

19. L'agent anti-TNF pour l'utilisation selon la revendication 17, dans lequel les au moins huit gènes comprennent CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEO ID NO: 32), et YIPF6 (SEQ ID NO: 36).

20. L'agent anti-TNF pour l'utilisation selon la revendication 16, dans lequel le sujet a été identifié par le fait d'avoir des niveaux d'expression élevés, en comparaison d'un individu sain, d'au moins 24 gènes, sélectionnés parmi l'ANKIB1 (SEQ ID NO: 1), et un ou plusieurs gènes supplémentaires sélectionnés parmi le groupe consistant en ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), et SFRS2 (SEQ ID NO: 32).

21. L'agent anti-TNF pour l'utilisation selon la revendication 20, dans lequel les au moins 24 gènes comprennent l'ANKIB1 (SEQ ID NO: 1) et de plus
ARF1 (SEQ 10 NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1 L (SEQ ID NO: 30), SERF2 (SEQ ID NO: 31), SFRS2 (SEQ ID NO: 32) et YIPF6 (SEQ ID NO: 36).

22. Le procédé selon l'une des revendications 1-15, dans lequel le niveau d'expression d'ANKIB1 (SEQ ID NO: 1) est élevé ou réduit, en comparaison d'un individu sain, par au moins environ 1,5 fois.

23. L'agent anti-TNF pour l'utilisation selon l'une des revendications 16-21, dans lequel le niveau d'expression d'ANKIB1 (SEQ ID NO: 1) est élevé en comparaison d'un individu sain, par au moins environ 1,5 fois.

24. Le procédé selon l'une des revendications 1-15, dans lequel le procédé comprend l'étape consistant à mesurer le niveau d'expression d'au moins cinq gènes sélectionnés parmi l'ANKIB1 (SEQ ID NO: 1) et un ou plusieurs gènes supplémentaires (i) sélectionnés parmi le groupe consistant en
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), et SFRS2 (SEQ IDNO: 32) ou (ii) sélectionnés parmi le groupe consistant en
ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1 L (SEQ ID NO: 30), SERF2 (SEQ ID NO: 31), SFRS2 (SEQ ID NO: 32) and YIPF6 (SEQ ID NO: 36),
ou (iii) comprenant cinq ou plusieurs de
CLTB (SEQ ID NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32), ou YIPF6 (SEQ ID NO: 36).

25. L'agent anti-TNF pour l'utilisation selon l'une des revendications 16-21, dans lequel le sujet a été identifié par le fait d'avoir des niveaux d'expression élevés, en comparaison d'un individu sain, d'au moins cinq gènes, sélectionnés parmi l'ANKIB1 (SEQ ID NO: 1), et un ou plusieurs gènes sélectionnés parmi le groupe consistant en
ARF1 (SEQ ID NO: 3), ARF5 (SEQ ID NO: 4), BRWD2 (SEQ ID NO: 5), CALM2 (SEQ ID NO: 7), CLTB (SEQ ID NO: 10), COL4A3BP (SEQ ID NO: 11), C9orf80 (SEQ ID NO: 6), EGLN2 (SEQ ID NO: 15), HDAC5 (SEQ ID NO: 19), LGALS9 (SEQ ID NO: 20), MYLIP (SEQ ID NO: 23), PCBP2 (SEQ ID NO: 24), PGK1 (SEQ ID NO: 25), RBBP4 (SEQ ID NO: 27), RER1 (SEQ ID NO: 28), RPA3 (SEQ ID NO: 29), SERF2 (SEQ ID NO: 31), SLC25A39 (SEQ ID NO: 33), SRGAP2 (SEQ ID NO: 34), TUG1 (SEQ ID NO: 35), YIPF6 (SEQ ID NO: 36), ZNF294 (SEQ ID NO: 38), ZFP36L1 (SEQ ID NO: 37), ANKRD12 (SEQ ID NO: 2), CAMK2G- (SEQ ID NO: 8), CASP5 (SEQ ID NO: 9), CXorf52 (SEQ ID NO: 12), DNAH1 (SEQ ID NO: 13), EEA1 (SEQ ID NO: 14), FAM44A (SEQ ID NO: 16), FOXJ3 (SEQ ID NO: 17), HDAC4 (SEQ ID NO: 18), MNT (SEQ ID NO: 21), MXRA7 (SEQ ID NO: 22), PTCH1 (SEQ ID NO: 26), SEL1L (SEQ ID NO: 30), et SFRS2 (SEQ ID NO: 32), (ii) sélectionnés parmi le groupe consistant en ARF1 (SEQ ID NO: 3), ARF5 (SEQ ID NO: 4), C9orf80 (SEQ ID NO: 6), CALM2 (SEQ ID NO: 7), CASP5 (SEQ ID NO: 9), CLTB (SEQ ID NO: 10), COL4A3BP (SEQ ID NO: 11), CXorf52 (SEQ ID NO: 12), DNAH1 (SEQ ID NO: 13), EEA1 (SEQ ID NO: 14), EGLN2 (SEQ ID NO: 15), FAM44A (SEQ ID NO: 16), HDAC4 (SEQ ID NO: 18), HDAC5 (SEQ ID NO: 19), LGALS9 (SEQ ID NO: 20), MXRA7 (SEQ ID NO: 22), PGK1 (SEQ ID NO: 25), RBBP4 (SEQ ID NO: 27), RER1 (SEQ ID NO: 28), SEL1 L (SEQ ID NO: 30), SERF2 (SEQ ID NO: 31), SFRS2 (SEQ ID NO: 32), et YIPF6 (SEQ ID NO: 36),ou (iii) comprenant cinq ou plusieurs de CLTB (SEQ 10 NO: 10), COL4A3BP (SEQ ID NO: 11), CXorf52 (SEQ ID NO: 12), FAM44A (SEQ ID NO: 16), MXRA7 (SEQ ID NO: 22), PGK1 (SEQ ID NO: 25), SFRS2 (SEQ ID NO: 32), ou YIPF6 (SEQ ID NO: 36).

26. Le procédé selon l'une des revendications 1-15, 22 ou 24, ou l'agent anti-TNF pour l'utilisation selon l'une des revendications 16-21, 23 ou 25, dans lequel le sujet a un désordre inflammatoire.

27. Le procédé selon l'une des revendications 1-15, 22 ou 24, ou l'agent anti-TNF pour l'utilisation selon l'une des revendications 16-21, 23 ou 25, dans lequel le sujet a l'arthrite rhumatoïde ou la maladie de Crohn.

28. Le procédé selon l'une des revendications 1-15, 22, 24, 26 ou 27 ou l'agent anti-TNF pour l'utilisation selon l'une des revendications 16-21, 23 ou 25-27, dans lequel le sujet est un homme.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6270766 B
- WO 06122187 A
- WO 2004096273 A
- EP 1536012 A
- WO 2006119115 A
- US 5559012 A
- US 5888510 A
- US 20010001663 A
- WO 0674399 A
- US 5656272 A
- US 5919452 A
- US 2004086915 A
- EP 0543942 A
- US 7101663 B
- US 6812341 B
- US 5445934 A
- US 6027880 A
- US 6057100 A
- US 6156501 A
- US 6261776 A
- US 6576424 A
- US 20030013208 A
- US 2004171068 A

- US 4582789 A
- US 4563417 A
- US 6193969 A
- US 6403087 B
- US 5925351 B
- US 6896885 B
- US 7060667 B
- US 5427779 A
- US 6197599 B
- US 5902723 B
- US 5871928 B
- US 5981180 A, Chandler
- US 5028545 A, Soini
- US 4499052 A, Fulwyler
- US 20040179267 A
- US 20040132205 A
- US 20040130786 A
- US 20040130761 A
- US 20040126875 A
- US 20040125424 A
- US 20040075907 A
- US 6916661 B

**Non-patent literature cited in the description**

- **SANDBORN et al.** *Gut,* 2002, vol. 53 (10), 1485-1493
- **CHOY et al.** *Rheumatology,* 2002, vol. 41 (10), 1133-1137
- **KAUSHIK et al.** *Expert Opinion on Biological Therapy,* 2005, vol. 5 (4), 601-606
- **SAMBROOK et al.** Molecular Cloning: A Laboratory Manual. Cold Spring Harbor Laboratory Press, November 1989, vol. 1, 2 3
- **GIBSON et al.** *Genome Res.,* 1999, vol. 6 (10), 995-1001
- **ZHANG et al.** *Environ. Sci. Technol.,* 2005, vol. 39 (8), 2777-2785
- Current Protocols in Immunology. Wiley, 1995
- **HAYMES et al.** Nucleic Acid Hybridization, A Practical Approach. IRL Press, 1985
- **AUSUBEL et al.** Current Protocols in Molecular Biology. John Wiley & Sons, 1989
- **AUSUBEL et al.** Current Protocols in Molecular Biology. John Wiley & Sons, 1999
- **HARLOW ; LANE.** Antibodies: A Laboratory Manual. Cold Spring Harbor Laboratory Press, 1988

- **HARLOW ; LANE.** Using Antibodies: A Laboratory Manual. Cold Spring Harbor Press, 1999
- Textbook of Clinical Chemistry. W.B. Saunders, 1999
- **BRAUN et al.** *Ann. Rheum. Dis.,* 2003, vol. 62, 1023-1024
- **FELSON et al.** *Arthritis and Rheumatism,* 1995, vol. 38 (6
- **LI et al.** *Genome Biol,* 2001, vol. 2
- **IRIZARRY et al.** *Nucleic Acids Res,* 2003, vol. 31, e15
- **WU et al.** *Journal of the American Statistical Association,* 2004, vol. 99, 909-917
- **SMYTH, G. K.** *Stat. Appl. Genet. Mol. Biol.,* 2004, vol. 3
- **BREIMAN, L.** *Machine Learning,* 2001, vol. 45, 5-32
- **FRANSEN et al.** *Clin Exp Rheumatol,* 2005, vol. 23, 93-99
- **SMYTH, G. K.** *Stat Appl Genet Mol Biol,* 2004, vol. 3

- **BRIEMAN.** *Machine Learning,* 2001, vol. 45, 5-32
- **GENTLEMAN et al.** Bioinformatics and Computational Biology Solutions using R and Bioconductor. New York. Springer
- **DIAZ-URIARTE et al.** *BMC Bioinformatics,* 2006, vol. 7 (3